(19) **Europäisches Patentamt / European Patent Office / Office européen des brevets**

(11) **EP 3 603 665 B1**

(12) **EUROPEAN PATENT SPECIFICATION**

(45) Date of publication and mention of the grant of the patent:
**31.12.2025  Bulletin 2026/01**

(51) International Patent Classification (IPC):
**A61K 39/00** *(2006.01)*   **A61K 35/15** *(2025.01)*
**A61K 35/17** *(2025.01)*   **A61K 39/395** *(2006.01)*
**C12Q 1/68** *(2018.01)*   **A61P 35/00** *(2006.01)*

(21) Application number: **19196027.7**

(22) Date of filing: **27.04.2016**

(52) Cooperative Patent Classification (CPC):
**C12Q 1/6886; A61K 40/11; A61K 40/42;
A61K 40/4201; A61P 35/00; A61P 37/04;
A61P 43/00; C12N 5/0636;** A61K 2239/31;
A61K 2239/38; A61K 2239/57; C12Q 2600/156

(54) **METHODS FOR PRODUCING PERSONALISED ENGINEERD T-CELLS FOR THE TREATMENT OF CANCER**

METHODEN ZUR HERSTELLUNG PERSONALISIERTER T-ZELLEN FÜR DIE BEHANDLUNG VON KREBS

MÉTHODES DE PRODUCTION DE CELLULES T PERSONNALISÉES POUR LE TRAITEMENT DU CANCER

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.04.2015  GB 201507100
02.03.2016  GB 201603663
03.03.2016  GB 201603731**

(43) Date of publication of application:
**05.02.2020  Bulletin 2020/06**

(62) Document number(s) of the earlier application(s) in accordance with Art. 76 EPC:
**16718705.3 / 3 288 581**

(73) Proprietor: **Cancer Research Technology Limited**
**London E20 1JQ (GB)**

(72) Inventors:
 • **MCGRANAHAN, Nicholas**
  **London, E20 1JQ (GB)**
 • **ROSENTHAL, Rachel**
  **London, NW1 1AT (GB)**
 • **SWANTON, Charles**
  **London, E20 1JQ (GB)**
 • **PEGGS, Karl**
  **London, E20 1JQ (GB)**
 • **QUEZADA, Sergio**
  **London, E20 1JQ (GB)**

(74) Representative: **Mewburn Ellis LLP
Aurora Building
Counterslip
Bristol BS1 6BX (GB)**

(56) References cited:
**WO-A1-2015/014375   WO-A2-2014/168874**

 • LI L ET AL: "Cancer genome sequencing and its implications for personalized cancer vaccines", CANCERS, vol. 3, no. 4, 25 November 2011 (2011-11-25), pages 4191 - 4211, XP002730781, ISSN: 2072-6694, DOI: 10.3390/CANCERS3044191
 • JAMAL-HANJANI M ET AL: "Tumour heterogeneity and immune-modulation", CURRENT OPINION IN PHARMACOLOGY, vol. 13, no. 4, August 2013 (2013-08-01), NL, pages 497 - 503, XP055283447, ISSN: 1471-4892, DOI: 10.1016/j.coph.2013.04.006
 • GERLINGER M ET AL: "Ultra-deep T cell receptor sequencing reveals the complexity and intratumour heterogeneity of T cell clones in renal cell carcinomas", JOURNAL OF PATHOLOGY, vol. 231, no. 4, 12 December 2013 (2013-12-12), GB, pages 424 - 432, XP055295760, ISSN: 0022-3417, DOI: 10.1002/path.4284

- N. MCGRANAHAN ET AL: "Clonal neoantigens elicit T cell immunoreactivity and sensitivity to immune checkpoint blockade", SCIENCE, vol. 351, no. 6280, 25 March 2016 (2016-03-25), pages 1463 - 1469, XP055283414, ISSN: 0036-8075, DOI: 10.1126/science.aaf1490
- YAP TIMOTHY A ET AL: "Intratumor Heterogeneity: Seeing the Wood for the Trees", SCIENCE TRANSLATIONAL MEDICINE, 28 March 2012 (2012-03-28), pages 127 - 10, XP093012555, Retrieved from the Internet <URL:https://www.science.org/doi/10.1126/scitranslmed.3003854> [retrieved on 20230110], DOI: 10.1126/scitranslmed.3003854
- LOHR JENS G ET AL: "Widespread Genetic Heterogeneity in Multiple Myeloma: Implications for Targeted Therapy", CANCER CELL, CELL PRESS, US, vol. 25, no. 1, 13 January 2014 (2014-01-13), pages 91 - 101, XP028809001, ISSN: 1535-6108, DOI: 10.1016/J.CCR.2013.12.015
- SCHUMACHER TON N. ET AL: "Neoantigens in cancer immunotherapy", SCIENCE, vol. 348, no. 6230, 3 April 2015 (2015-04-03), US, pages 69 - 74, XP055866872, ISSN: 0036-8075, DOI: 10.1126/science.aaa4971
- CHRISTIAN S HINRICHS ET AL: "Exploiting the curative potential of adoptive T-cell therapy for cancer", IMMUNOLOGICAL REVIEWS, WILEY-BLACKWELL PUBLISHING, INC, US, vol. 257, no. 1, 13 December 2013 (2013-12-13), pages 56 - 71, XP071455752, ISSN: 0105-2896, DOI: 10.1111/IMR.12132
- LILY ZHENG: "Estimation of cancer cell fractions and clone trees from multi-region sequencing of tumors", BIOINFORMATICS, vol. 38, no. 15, 1 June 2022 (2022-06-01), GB, pages 3677 - 3683, XP093157574, ISSN: 1367-4803, Retrieved from the Internet <URL:https://academic.oup.com/bioinformatics/article-pdf/38/15/3677/49884133/btac367.pdf> DOI: 10.1093/bioinformatics/btac367
- MAREK CMERO: "Inferring structural variant cancer cell fraction", NATURE COMMUNICATIONS, vol. 11, no. 1, 5 February 2020 (2020-02-05), UK, pages 1 - 15, XP93157570, ISSN: 2041-1723, Retrieved from the Internet <URL:https://www.nature.com/articles/s41467-020-14351-8> DOI: 10.1038/s41467-020-14351-8

**Description**

FIELD OF THE INVENTION

The present invention relates to methods and compositions which are useful for the treatment of cancer. In particular, the present invention relates to a method for producing a T cell composition comprising an engineered T cell, wherein the T cell is engineered to express an introduced T cell receptor (TCR) which specifically binds a clonal neoantigen from a subject's cancer.

BACKGROUND TO THE INVENTION

It is known that intra-tumoural heterogeneity (ITH) and the mutational landscape of a tumour can influence the ability of the immune system to respond to cancer.

For instance, genetic instability plays a major role in the ability of tumour cells to develop escape mutants that evade immune elimination. This fundamental characteristic of tumour cells is a major reason why many promising immunotherapies designed to elicit potent tumour antigen-specific T cell immunity ultimately fail, and it poses a considerable challenge in the development of successful cancer vaccine strategies. As such, immunotherapies designed to establish antigen-specific T cell immunity against tumours present a paradox in that tumour-specific immunity that effectively eliminates the tumour also applies selective pressure that promotes the development of tumour escape mutants that are resistant to T cell elimination. Numerous reports indicate that tumours escape immune elimination by the selective growth of tumour cells expressing random mutations that either initiate or silence genes through point mutations, frame-shift mutations, genomic translocations, insertions, or deletions.

Tumour heterogeneity describes the observation that different tumour cells can show distinct morphological and phenotypic profiles, including differences in cellular morphology, gene expression, genetic and epigenetic mutations, metabolism, motility, proliferation, and metastatic potential. This phenomenon occurs both between tumours (inter-tumour heterogeneity) and within tumours (intratumour heterogeneity or ITH). The heterogeneity of cancer cells presents significant challenges in designing effective treatment strategies.

By way of example, heterogeneous tumours may exhibit different sensitivities to cytotoxic or targeted drugs among different clonal populations. This is attributed to clonal interactions that may inhibit or alter therapeutic efficacy, posing a challenge for successful therapies in heterogeneous tumours (and their heterogeneous metastases).

Drug administration in heterogeneous tumours will seldom kill all tumour cells. The initial heterogeneous tumour population may bottleneck, such that few drug resistant cells will survive. This allows resistant tumour populations to replicate and re-grow the tumour through a branching evolution mechanism. The resulting repopulated tumour may also be heterogeneous and will be resistant to the initial drug therapy used. The repopulated tumour may also return in a more aggressive manner.

The administration of cytotoxic drugs often results in initial tumour shrinkage. This represents the destruction of initial non-resistant subclonal populations within a heterogeneous tumour, leaving only resistant clones. These resistant clones now contain a selective advantage in the presence of chemotherapy and can replicate to repopulate the tumour. Replication will likely occur through branching evolution, contributing to tumour heterogeneity. The repopulated tumour may appear to be more aggressive. This is attributed to the drug-resistant selective advantage of the tumour cells and additional genetic changes that occur during therapy and the disease course.

WO 2014/168874 describes a method for making a personalized neoplastic vaccine for a subject diagnosed as having a neoplasia, which includes identifying a plurality of mutations in the neoplasia, analysing the plurality of mutations to identify a subset of at least five neo-antigenic mutations predicted to encode neo-antigenic peptides and producing, based on the identified subset, a personalised neoplasia vaccine.

Thus there is a need for alternative methods for treating cancer, in particular heterogeneous tumours.

SUMMARY OF ASPECTS OF THE INVENTION

The present inventors have determined that truncal mutations, that is mutations present in essentially all tumour cells in a heterogeneous tumour, can be identified through multi-region sampling of the tumour or through approaches to identify clonal mutations in single biopsies. For example, the cancer cell fraction, describing the fraction of cancer cells harbouring a mutation, can be determined in order to distinguish neo-antigens likely to be present in every cancer cell in the tumour (truncal neo-antigens) from neo-antigens only present in a subset of tumour cells (branch neo-antigens). As used herein, the term "truncal mutations" is synonymous with the term "clonal mutations". They are both intended to define mutations present in essentially all tumour cells in a heterogeneous tumour. As used herein, the term "branched mutations" is synonymous with the term "sub-clonal mutations". They are both intended to define mutations present in a subset of tumour cells. The administration of therapeutic T cells which target truncal neo-antigens, rather than branch neo-antigens, or the administration of vaccines as described herein, enables an effective immune response to be mounted against the entire tumour and thus reduces the risk of resistant cells repopulating the tumour.

The invention provides a method for producing a T cell composition comprising an engineered T cell, wherein the T cell is engineered to express an introduced T cell receptor (TCR) which specifically binds a clonal neoantigen from a subject's cancer, wherein the method comprises the following steps:

(a) identifying a clonal neoantigen-specific T cell from the subject which comprises the steps of:

i) determining mutations present in one or more samples isolated from the subject's tumour;
ii) identifying a clonal mutation, wherein a clonal mutation has a cancer cell fraction (CCF) estimate with a 95% confidence interval greater than or equal to 0.75 in each sample, wherein the CCF estimate in a sample is calculated by integrating variant allele frequencies, copy numbers and purity estimates for the sample;
iii) identifying a clonal neoantigen, which is an antigen encoded by a sequence which comprises the clonal mutation;
vi) identifying a T cell from a sample isolated from the subject which is capable of specifically recognising the clonal neoantigen as a clonal neoantigen-specific T cell;

(b) isolating a TCR gene that encodes a TCR from the clonal neoantigen-specific T cell; and
(c) engineering a T cell to express said TCR which recognises said clonal neoantigen to provide a T cell population which targets the clonal neoantigen; and
(d) expanding the engineered T cell to provide a T cell composition.

The invention also provides a T cell composition for use in treating cancer in the subject, wherein the treatment comprises the method as defined above.

A method for identifying a truncal neo-antigen in a tumour from a subject may comprise the steps of:

i) determining mutations present in a sample isolated from the tumour; and
ii) identifying a truncal mutation which is a mutation present in essentially all tumour cells; and
iii) identifying a truncal neo-antigen, which is an antigen encoded by a sequence which comprises the truncal mutation.

A method for identifying a truncal neo-antigen in a tumour from a subject may comprise the steps of:

i) determining mutations present in a plurality of samples isolated from the tumour; and
ii) identifying a truncal mutation which is a mutation present in all samples; and
iii) identifying a truncal neo-antigen, which is an antigen encoded by a sequence which comprises the truncal mutation.

The truncal mutation may be a single nucleotide variant or an insertion/deletion or a splice site mutation resulting in a change in the amino acid sequence (coding mutation).

The mutations may be identified by Exome sequencing, RNA-seq, whole genome sequencing and/or targeted gene panel sequencing. Suitable methods are known in the art. Descriptions of Exome sequencing and RNA-seq are provided by Boa et al. (Cancer Informatics. 2014;13(Suppl 2):67-82.) and Ares et al. (Cold Spring Harb Protoc. 2014 Nov 3;2014(11):1139-48); respectively. Descriptions of targeted gene panel sequencing can be found in, for example, Kammermeier et al. (J Med Genet. 2014 Nov;51(11):748-55) and Yap KL et al. (Clin Cancer Res. 2014. 20:6605). See also Meyerson et al., Nat. Rev. Genetics, 2010 and Mardis, Annu Rev Anal Chem, 2013. Targeted gene sequencing panels are also commercially available (e.g. as summarised by Biocompare (http://www.biocompare.com/Editorial-Articles/161194-Build-Your-Own-Gene-Panels-with-These-Custom-NGS-Targeting-Tools/).

The method may comprise the step of assessing the subject's HLA allele profile to determine if a truncal neo-antigen peptide will bind to a MHC molecule of the subject. Suitable methods are known in the art, e.g. OptiType, Szolek et al., 2014.

A method for providing a T cell population which targets a truncal neo-antigen in a tumour from a subject may comprise the steps of:

i) identifying a T cell from a sample isolated from the subject, which is capable of specifically recognising a truncal neo-antigen peptide; and
ii) expanding the T cell to provide a T cell population which targets the truncal neoantigen.

One skilled in the art will appreciate that references herein to a T cell "recognising" a truncal neo-antigen or truncal neo-antigen peptide include recognition in the form of a truncal neo-antigen peptide:MHC complex.

A method for identifying a truncal neo-antigen specific T cell may comprise the following steps:

i) determining mutations present in a sample isolated from the tumour; and
ii) identifying a truncal mutation which is a mutation present in essentially all tumour cells; and
iii) identifying a truncal neo-antigen, which is an antigen encoded by a sequence which comprises the truncal mutation;
or

i) determining the mutations present in a plurality of samples isolated from a tumour;
ii) identifying a truncal mutation which is a mutation present in all samples;
iii) identifying a truncal neo-antigen, which is an antigen encoded by a sequence which comprises the truncal mutation;
and
iv) identifying from a sample from said subject a T cell capable of specifically recognising the truncal neo-antigen as a truncal neo-antigen specific T cell.

The sample from which the T cell is identified may be a blood sample, a tumour sample, a tumour-associated lymph node sample or sample from a metastatic site.
A method for providing a T cell population which targets a truncal neo-antigen in a tumour may comprise the steps of:

(a) identifying a truncal neo-antigen in a tumour from a subject which comprises the steps of:

i) determining mutations present in a sample isolated from the tumour; and
ii) identifying a truncal mutation which is a mutation present in essentially all tumour cells; and
iii) identifying a truncal neo-antigen, which is an antigen encoded by a sequence which comprises the truncal mutation;
or

i) determining the mutations present in a plurality of samples isolated from a tumour;
ii) identifying a truncal mutation which is a mutation present in all samples;
iii) identifying a truncal neo-antigen, which is an antigen encoded by a sequence which comprises the truncal mutation; and

b) identifying a T cell from a sample isolated from a subject which is capable of specifically recognising said truncal neo-antigen; and
c) expanding the T cell to provide a T cell population which targets the truncal neo-antigen.

Described herein is a T cell population which is obtained or obtainable by a method which comprises the steps of:

(a) identifying a truncal neo-antigen in a tumour from a subject which comprises the steps of:

i) determining mutations present in a sample isolated from the tumour; and
ii) identifying a truncal mutation which is a mutation present in essentially all tumour cells; and
iii) identifying a truncal neo-antigen, which is an antigen encoded by a sequence which comprises the truncal mutation;
or

i) determining the mutations present in a plurality of samples isolated from a tumour;
ii) identifying a truncal mutation which is a mutation present in all samples;
iii) identifying a truncal neo-antigen, which is an antigen encoded by a sequence which comprises the truncal mutation; and

b) identifying a T cell from a sample isolated from a subject which is capable of specifically recognising said truncal neo-antigen; and
c) expanding the T cell to provide a T cell population which targets the truncal neo-antigen.

Thus, the resulting T cell population is enriched with an increased number of T cells which target truncal neo-antigens (for example, compared with the sample isolated from the subject).
The sample may be a tumour, blood, tissue or peripheral blood mononuclear cells from the subject.

The truncal neo-antigen may be generated by a truncal mutation identified according to the methods described herein.

The population of T cells may comprise CD8$^+$ T cells, CD4$^+$ T cells or CD8$^+$ and CD4$^+$ T cells.

The method may comprise providing at least a first and a second T cell, wherein the first T cell targets a first truncal neo-antigen generated by a first truncal mutation and the second T cell targets a second truncal neo-antigen generated by a second truncal mutation.

Described herein is a T cell composition which comprises a truncal neo-antigen specific T cell or a population of T cells as described herein.

The truncal neo-antigen may be identified by the methods described herein.

The truncal neo-antigen specific T cell may express a chimeric antigen receptor (CAR) or a T cell receptor (TCR) or an affinity-enhanced T cell receptor (TCR) which specifically binds a truncal neo-antigen or truncal neo-antigen peptide (i.e. a peptide derived from the truncal neo-antigen), as discussed further hereinbelow.

Methods for generating TCRs and affinity enhanced TCRs are known in the art. Affinity enhanced TCRs are TCRs with enhanced affinity for a peptide-MHC complex. Methods include e.g. the isolation of TCR genes that encode TCRs from patient samples (e.g. patient peripheral blood or TILs), and the improvement of TCR affinity for a peptide-MHC complex via modification of TCR sequences (e.g. by *in vitro* mutagenesis and selection of enhanced affinity (or affinity matured) TCRs). Methods of introducing such TCR genes into T cells are known in the art. Methods of identifying optimal-affinity TCRs involving the immunisation of antigen-negative humanised transgenic mice which have a diverse human TCR repertoire (e.g. TCR/MHC humanised mice such as ABabDII mice) with antigen, and isolation of antigen-specific TCRs from such immunised transgenic mice are also known in the art (see e.g. Obenaus M et al., Nat Biotechnol. 33(4):402-7, 2015).

Described herein is an MHC multimer comprising a truncal neo-antigen peptide, wherein the truncal neo-antigen is identified by the methods described herein. MHC multimers and methods of using them to isolate T cells are known in the art, for example as described in Hadrup, Nature Methods 6:520-526 2009; and Andersen, Nature Protocol 7:891-902, 2012.

MHC multimers as described herein may be used in methods of the invention, for example in methods of identifying NES T cells. The MHC multimers may be used to identify, expand or enrich NES T cells in methods as described herein, for example methods for producing a T cell or T cell population or composition as described herein.

Described herein is a vaccine comprising a truncal neo-antigen peptide from a truncal neo-antigen identified by the methods described herein. As discussed herein, a truncal neo-antigen vaccine may be delivered as a dendritic cell vaccine pulsed or loaded with the truncal neo-antigen, or genetically modified (via DNA or RNA transfer) to express one, two or more truncal neo-antigens.

Described herein is a T cell as defined herein for use in the manufacture of a medicament for the treatment of cancer.

A method for treating cancer in a subject may comprise administering a T cell composition to the subject.

The method may comprise the following steps:

    (i) isolation of a T cell containing sample from the subject;
    (ii) identification and expansion of a T cell population which targets the truncal neo-antigen; and
    (iii) administering the cells from (ii) to the subject.

The method may comprise the following steps:

    (i) isolation of a T cell containing sample;
    (ii) engineering the T cell to express a CAR or TCR which recognises said truncal neo-antigen as described herein to provide a T cell population which targets the truncal neo-antigen; and
    (iii) administering the cells from (ii) to the subject.

The method may also encompass the step of identifying a truncal neo-antigen as described herein, i.e. a method for treating cancer in a subject may comprise;

    (a) identifying a truncal neo-antigen in a tumour from a subject which comprises the steps of:

        i) determining mutations present in a sample isolated from the tumour; and
        ii) identifying a truncal mutation which is a mutation present in essentially all tumour cells; and
        iii) identifying a truncal neo-antigen, which is an antigen encoded by a sequence which comprises the truncal mutation;
        or

i) determining the mutations present in a plurality of samples isolated from a tumour;
ii) identifying a truncal mutation which is a mutation present in all samples;
iii) identifying a truncal neo-antigen, which is an antigen encoded by a sequence which comprises the truncal mutation; and

b) identifying a T cell from a sample isolated from a subject which is capable of specifically recognising said truncal neo-antigen;
c) expanding the T cell to provide a T cell population which targets the truncal neoantigen; and
d) administering said T cell population to said subject.

The method may comprise the steps of;

(a) identifying a truncal neo-antigen in a tumour from a subject which comprises the steps of:

i) determining mutations present in a sample isolated from the tumour; and
ii) identifying a truncal mutation which is a mutation present in essentially all tumour cells; and
iii) identifying a truncal neo-antigen, which is an antigen encoded by a sequence which comprises the truncal mutation;
or

i) determining the mutations present in a plurality of samples isolated from a tumour;
ii) identifying a truncal mutation which is a mutation present in all samples;
iii) identifying a truncal neo-antigen, which is an antigen encoded by a sequence which comprises the truncal mutation; and

(b) providing a T cell containing sample;
(c) engineering a T cell to express a CAR or TCR which recognises said truncal neo-antigen to provide a T cell population which targets the truncal neoantigen; and
(d) administering said T cell population to the subject.

The T cell may be engineered to express a CAR or affinity-enhanced TCR as described herein.
A method of treating a patient who has cancer may comprise:

(i) identifying a patient who has cancer; and
(ii) administering to said patient a T cell or T cell population as defined herein.

The truncal neo-antigen, T cell or T cell population may have been identified or produced as described herein.
The sample may be a tumour sample, a blood sample or a tissue sample or a peripheral blood mononuclear cells sample from the subject.
A method for treating cancer in a subject may comprise administering a vaccine as described herein to the subject.

DESCRIPTION OF THE FIGURES

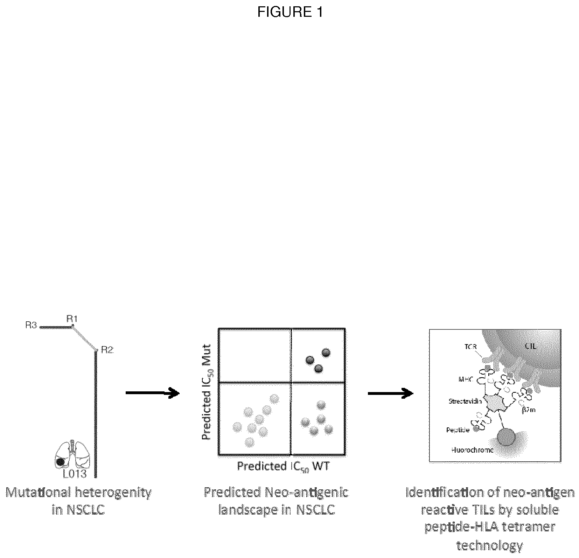

Figure 1 - Pipeline for prediction and identification of neo-antigen reactive T cells in NSCLC samples. Exome seq and RNAseq are used to define neo-epitopes from truncal and branch mutations. The binding of mutant or wild type peptides to the patient's HLA is predicted and peptides with predicted high affinity (green) to HLA (low $IC_{50}$) and those with high affinity as mutant and low as a wild type (blue) are selected to generate fluorescent MHC multimers to be used in the identification of NES T cells in tumour samples.

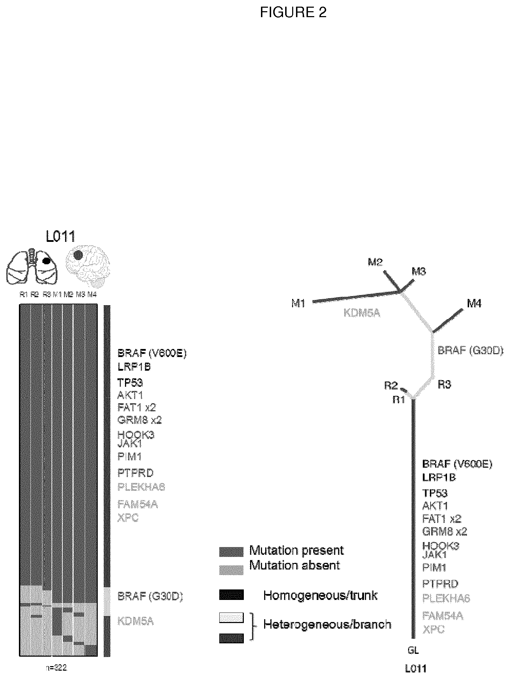

**Figure 2** - Illustration of the difference between truncal mutations and branch mutations in sample isolated from lung cancer, brain cancer and normal lung and brain tissue.

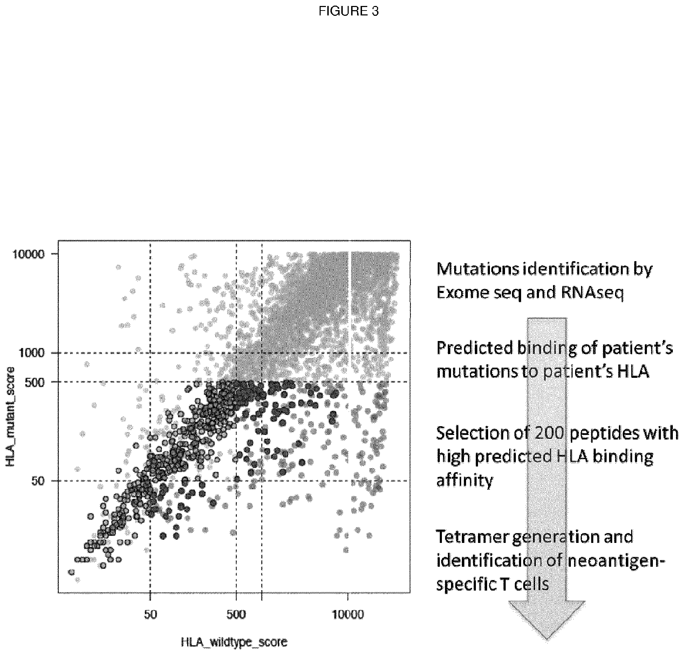

**Figure 3** - Illustration of the pipeline from identification of truncal mutations in a tumour to the identification of neo-antigen-specific T cells.

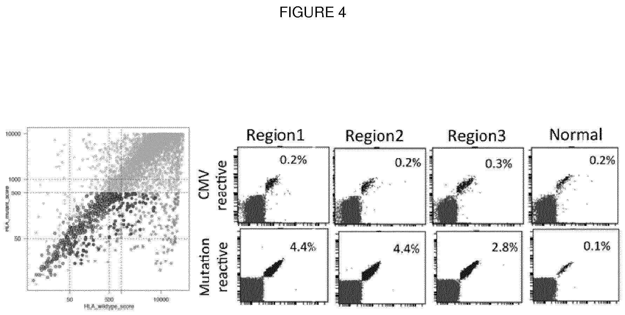

**Figure 4** - **(A)** Identification of neo-antigen specific T cells in early stage lung cancer. Predicted affinities of mutant (Y axis) versus wild type (X axis) obtained from exon sequencing data are shown. Red dots indicate predictive peptides with a high score (low affinity) for the patient's HLA in the WT form and low score (high affinity) in the mutant form. (B) *In vitro* expanded TILs were stained with fluorescent tetramers loaded with the predicted mutated peptides or control Cytomegalovirus (CMV) peptides and analyzed by flow cytometry. CMV reactive T cells are found at equal frequencies (0.2-0.3%) in tumour and normal lung.

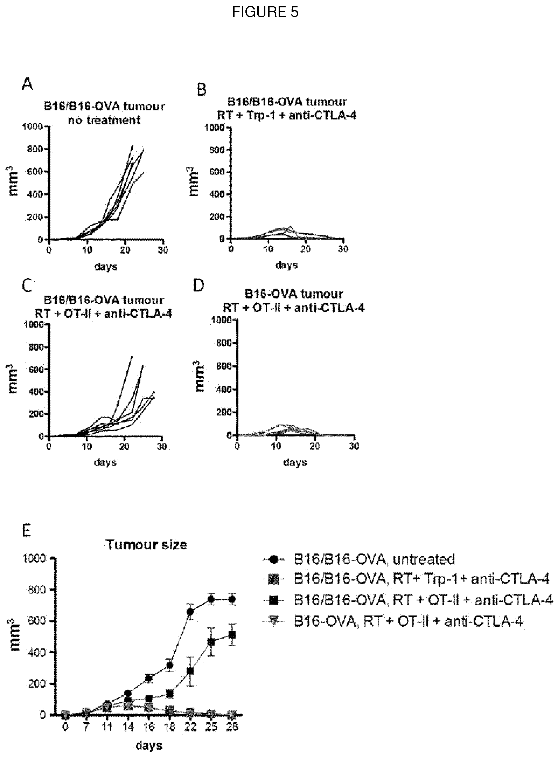

**Figure 5** - (A) mice challenged with the heterogenous tumour mix (B16/B16-OVA) containing a clonal (tyrp1) and subclonal (OVA) neo-antigen and left untreated grew tumours and had to be sacrificed between days 20 and 30 after tumour challenge (B) mice challenged with the B16/B16-OVA tumour mix but treated with TRP1 TCR Tg cells targeting a clonal neo-antigen were able to reject their tumours. (C) mice were treated with OTII TCR Tg T cells targeting a subclonal neo-antigen, none of the mice were able to reject their tumour. (D) demonstrates the ability of OTII TCRTg T cells to reject established tumours when all cells in the tumour express the OVA neo-antigen. Each line in each graph represents an independent mouse. 6 mice per groups were used for these experiments. (E) shows all the experimental groups and the average tumour size in each group.

DETAILED DESCRIPTION OF THE INVENTION

TRUNCAL NEO-ANTIGEN

The present invention involves a method for identifying truncal (clonal) neo-antigens in a tumour.

A 'neo-antigen' is a tumour-specific antigen which arises as a consequence of a mutation within a cancer cell. Thus, a neo-antigen is not expressed by healthy cells in a subject. As such, one advantage of targeting a truncal neo-antigen therapeutically is lower levels of predicted toxicity because healthy cells are not targeted.

Many antigens expressed by cancer cells are self-antigens which are selectively expressed or over-expressed on the cancer cells. These self-antigens are difficult to target with cellular immunotherapy because they require overcoming both central tolerance (whereby autoreactive T cells are deleted in the thymus during development) and peripheral tolerance (whereby mature T cells are suppressed by regulatory mechanisms).

These tolerance mechanisms may be abrogated by the targeting of neo-antigens. In particular, non-silent mutations which occur in cancer cells can result in the expression of proteins by the cancer cell which are not expressed by healthy cells. These altered proteins are not recognised as 'self-antigens' by the immune system.

Because neo-antigens are not recognised as 'self-antigens', T cells which are capable of targeting neo-antigens are not subject to central and peripheral tolerance mechanisms to the same extent as T cells which recognise self-antigens.

The neo-antigen described herein may be caused by any non-silent mutation which alters a protein expressed by a cancer cell compared to the non-mutated protein expressed by a wild-type, healthy cell.

A 'mutation' refers to a difference in a nucleotide sequence (e.g. DNA or RNA) in a tumour cell compared to a healthy cell from the same individual. The difference in the nucleotide sequence can result in the expression of a protein which is not expressed by a healthy cell from the same individual.

For example, the mutation may be a single nucleotide variant (SNV), multiple nucleotide variants, a deletion mutation, an insertion mutation, a translocation, a missense mutation or a splice site mutation resulting in a change in the amino acid sequence (coding mutation). It is known that genome doubling can occur in cancer cells. A mutation which occurs before a genome doubling event will therefore be present in a cancer cell at twice the relative copy number of a mutation which occurred after the doubling event. If the Genome doubling event is a truncal event present in every cell, neo-antigens occurring before genome doubling would represent a preferential neo-antigenic target for the reasons stated. In a preferred embodiment the truncal neo-antigen is one present in a region of the genome that is rarely subject to copy number loss.

In particular embodiments, the mutation which produces the neo-antigen is a SNV.

Different regions of tumours may be morphologically distinct. In addition, intratumour mutational heterogeneity may occur and can be associated with differences in tumour prognosis and the potential ability of tumour cells to escape immune therapies targeting mutations which are not present in all or most tumour cells.

The present inventors have determined that intratumour heterogeneity can cause variation between the neo-antigens expressed in different regions of a tumour and between different cells in a tumour. In particular, the inventors have determined that, within a tumour, certain neo-antigens are expressed in all regions and essentially all cells of the tumour whilst other neo-antigens are only expressed in a subset of tumour regions and cells.

As such, a "truncal" or "clonal" neo-antigen is a neo-antigen which is expressed effectively throughout a tumour and encoded within essentially every tumour cell. A "branch" or "sub-clonal" neo-antigen' is a neo-antigen which is expressed in a subset or a proportion of cells or regions in a tumour.

'Present throughout a tumour', 'expressed effectively throughout a tumour' and 'encoded within essentially every tumour cell' may mean that the truncal neo-antigen is expressed in all regions of the tumour from which samples are analysed.

It will be appreciated that a determination that a mutation is 'encoded within essentially every tumour cell' refers to a statistical calculation and is therefore subject to statistical analysis and thresholds.

Likewise, a determination that a truncal neo-antigen is 'expressed effectively throughout a tumour' refers to a statistical calculation and is therefore subject to statistical analysis and thresholds.

Expressed effectively in essentially every tumour cell or essentially all tumour cells means that the mutation is present in all tumour cells analysed in a sample, as determined using appropriate statistical methods.

By way of the example, the cancer cell fraction (CCF), describing the proportion of cancer cells that harbour a mutation may be used to determine whether mutations are truncal or branched. For example, the cancer cell fraction may be determined by integrating variant allele frequencies with copy numbers and purity estimates as described by Landau et al. (Cell. 2013 Feb 14;152(4):714-26). A determination of the CCF is demonstrated in the Examples described herein.

In brief, CCF values are calculated for all mutations identified within each and every tumour region analysed. If only one region is used (i.e. only a single sample), only one set of CCF values will be obtained. This will provide information as to which mutations are present in all tumour cells within that tumour region, and will thereby provide an indication if the mutation is truncal or branched. All sub clonal mutations (i.e. CCF<1) in a tumour region are determined as branched, whilst clonal mutations with a CCF=1 are determined to be truncal.

As stated, determining a truncal mutation is subject to statistical analysis and threshold. As such, a mutation may be identified as truncal if it is determined to have a CCF 95% confidence interval >= 0.75, for example 0.80, 0.85, 0.90, 0.95, 1.00 or >1.00. Conversely, a mutation may be identified as branched if it is determined to have a CCF 95% confidence interval <= 0.75, for example 0.70, 0.65, 0.60, 0.55, 0.50, 0.45, 0.40, 0.35, 0.30, 0.25, 0.20, 0.15, 0.10, 0.05, 0.01 in any sample analysed.

It will be appreciated that the accuracy of a method for identifying truncal mutations is increased by identifying clonal mutations for more than one sample isolated from the tumour.

In one embodiment the methods may involve identifying a plurality i.e. more than one clonal neo-antigen.

In one embodiment the number of clonal neo-antigens is 2-1000. For example, the number of clonal neo-antigens may be 2, 3, 4, 5, 6, 7, 8, 9, 10, 20, 50, 100, 150, 200, 250, 300, 350, 400, 450, 500, 550, 600, 650, 700, 750, 800, 850, 900, 950 or 1000, for example the number of clonal neo-antigens may be from 2 to 100.

In a preferred embodiment the method may provide a plurality or population, i.e. more than one, of T cells wherein the plurality of T cells comprises a T cell which recognises a clonal neo-antigen and a T cell which recognises a different clonal neoantigen. As such, the method provides a plurality of T cells which recognise different clonal neo-antigens.

In a preferred embodiment the number of clonal neo-antigens recognised by the plurality of T cells is 2-1000. For example, the number of clonal neo-antigens recognised may be 2, 3, 4, 5, 6, 7, 8, 9, 10, 20, 50, 100, 150, 200, 250, 300, 350, 400, 450, 500, 550, 600, 650, 700, 750, 800, 850, 900, 950 or 1000, for example the number of clonal neo-antigens recognised may be from 2 to 100.

In one aspect a plurality of T cells recognises the same truncal neo-antigen.

TUMOUR SAMPLES

The method comprises the step of determining the mutations present in essentially all cancer cells isolated from a tumour. References herein to "essentially all" are intended to encompass the majority of tumour cells in a subject. For example, this may comprise 60-100% of cells, e.g. 60, 61, 62, 63, 64, 65, 66, 67, 68, 69, 70, 71, 72, 73, 74, 75, 76, 77, 78, 79 80, 81, 82, 83, 84, 85, 86, 87, 88, 89, 90, 91, 92, 93, 94, 95, 96, 97, 98, 99 or 100% of tumour cells in a subject.

Isolation of biopsies and samples from tumours is common practice in the art and may be performed according to any suitable method and such methods will be known to one skilled in the art.

The tumour may be a solid tumour or a non-solid tumour.

The method of the present invention may comprise, for example, determining the mutations present in cancer cells from one or more tumour regions isolated from a tumour.

For example, the method may comprise determining the mutations present in at least two, at least three, at least four, at least five, at least six, at least seven, at least eight, at least nine or at least ten or more biopsies isolated from a tumour. The method can also be used to determine trunk (truncal) mutations in one biopsy.

The individual tumour samples may be isolated from different regions located throughout a tumour within a primary site or between primary and metastases or within a metastasis or between metastases. For example, determining the mutations present in tumours which are known to display morphological disparate histology in different regions may involve determining the mutations present in a number of individual samples isolated from morphologically disparate regions.

The sample may be a blood sample. For example, the blood sample may comprise circulating tumour DNA, circulating tumour cells or exosomes comprising tumour DNA.

Determining mutations present in a tumour sample may be performed by comparing DNA and/or RNA sequences isolated from tumour samples and comparative healthy samples from the same subject by Exome Sequencing, whole genome sequencing, targeted gene panel sequencing and/or RNA-Seq, for example. Descriptions of Exome sequencing and RNA-seq are provided by Boa *et al.* (Cancer Informatics. 2014;13(Suppl 2):67-82.) and Ares *et al.* (Cold Spring Harb Protoc. 2014 Nov 3;2014(11):1139-48); respectively.

Sequence alignment to identify nucleotide differences (e.g. SNVs) in DNA and/or RNA from a tumour sample compared to DNA and/or RNA from a non-tumour sample may be performed using methods which are known in the art. For example, nucleotide differences compared to a reference sample may be performed using the method described by Koboldt et al. (Genome Res.; 2012; 22: 568-576). The reference sample may be the germline DNA and/or RNA sequence.

HLA ALLELES

T cells which specifically recognise a neo-antigen are referred to herein as neoantigen specific (NES) T cells.

Antigens are presented to T cells in the context of antigen-derived peptides bound by major histocompatibility molecules (MHC).

Thus a truncal neo-antigen may be recognised by a NES T cell as a truncal neoantigen derived peptide (referred to herein as a 'truncal neo-antigen peptide') presented by an MHC molecule.

A truncal neo-antigen peptide is a peptide which is derived from the region of a polypeptide which comprises a cancer cell specific mutation. As such truncal neoantigen peptides should not be derived from polypeptides encoded by the genome of healthy cells.

MHC class I proteins form a functional receptor on most nucleated cells of the body. There are 3 major MHC class I genes in HLA: HLA-A, HLA-B, HLA-C and three minor genes HLA-E, HLA-F and HLA-G. $\beta$2-microglobulin binds with major and minor gene subunits to produce a heterodimer.

Peptides that bind to MHC class I molecules are typically 7 to 13, more usually 8 to 11 amino acids in length. The binding of the peptide is stabilised at its two ends by contacts between atoms in the main chain of the peptide and invariant sites in the peptide-binding groove of all MHC class I molecules. There are invariant sites at both ends of the groove which bind the amino and carboxy termini of the peptide. Variations in peptide length are accommodated by a kinking in the peptide backbone, often at proline or glycine residues that allow the required flexibility.

There are 3 major and 2 minor MHC class II proteins encoded by the HLA. The genes of the class II combine to form heterodimeric ($\alpha\beta$) protein receptors that are typically expressed on the surface of antigen-presenting cells.

Peptides which bind to MHC class II molecules are typically between 8 and 20 amino acids in length, more usually between 10 and 17 amino acids in length, and can be longer (for example up to 40 amino acids). These peptides lie in an extended conformation along the MHC II peptide-binding groove which (unlike the MHC class I peptide-binding groove) is open at both ends. The peptide is held in place mainly by main-chain atom contacts with conserved residues that line the peptide-binding groove.

The methods of the present invention may involve the step of assessing a subject's HLA alleles to determine if a truncal neo-antigen peptide will bind to an MHC molecule expressed by the subject.

The HLA allele profile of an individual may be determined by methods which are known in the art. For example, the HLA profile of an individual may be determined by HLA-serotyping and/or HLA gene sequencing. HLA-phenotyping with single specific primer-PCR (SSP-PCR) is an alternative strategy for determining the HLA profile of an individual.

In the present examples, the HLA profile of an individual is determined by sequencing of the HLA locus and processing using the Optitype prediction algorithm to determine the HLA type for each individual (Szolek et al.; Bioinformatics; 2014; 30(23):3310-3316).

The binding of a peptide to a particular MHC molecule may be predicted using methods which are known in the art. Examples of methods for predicting MHC binding include those described by Lundegaard et al. (Nucleic Acids Res. 2008:W509-12.2008 & Bioinformatics. 2008 Jun 1;24(11):1397-8) and Shen et al. (Proteome Sci. 2013 Nov 7;11(Suppl 1):S15).

The methods of the present invention may comprise determining a truncal neoantigen peptide which is predicted to bind to an MHC molecule expressed by the subject. In particular, the methods may comprise the step of determining and selecting a truncal neo-antigen peptide which is predicted to bind strongly to an MHC molecule expressed by the subject. The exact definition of 'binding strongly' will depend on the method used to predict the MHC binding interaction (see Lundegaard *et al.* (as above), for example). However, in all cases the truncal neo-antigen peptide selected will be predicted to be capable of binding to, and being presented in the context of, an MHC molecule expressed by the subject.

The binding affinity to a truncal neo-antigen peptide may be below 500nM. By "high affinity" may mean 0 to 50nM binding affinity. In other embodiments the truncal neoantigen peptide may bind the MHC molecule with an intermediate affinity of 50 to150nM binding affinity, or low affinity of 150 to 500nM binding affinity.

In certain embodiments, the truncal neo-antigen peptide may be predicted to bind to the MHC molecule with a high affinity whilst a corresponding wild-type peptide (e.g. an equivalent peptide derived from the same region of the corresponding wild-type polypeptide) is predicted to bind to the same MHC molecule with low affinity.

T CELL POPULATION

The present invention also relates to a method for providing a T cell population which targets a truncal neo-antigen from a tumour.

The T cell population may comprise CD8+ T cells, CD4+ T cells or CD8+ and CD4+ T cells.

Helper T helper cells (TH cells) assist other white blood cells in immunologic processes, including maturation of B cells into plasma cells and memory B cells, and activation of cytotoxic T cells and macrophages. TH cells express CD4 on their surface. TH cells become activated when they are presented with peptide antigens by MHC class II molecules on the surface of antigen presenting cells (APCs). These cells can differentiate into one of several subtypes, including TH1, TH2, TH3, TH17, Th9, or TFH, which secrete different cytokines to facilitate different types of immune responses.

Cytotoxic T cells (TC cells, or CTLs) destroy virally infected cells and tumour cells, and are also implicated in transplant rejection. CTLs express the CD8 at their surface. These cells recognize their targets by binding to antigen associated with MHC class I, which is present on the surface of all nucleated cells. Through IL-10, adenosine and other molecules secreted by regulatory T cells, the CD8+ cells can be inactivated, which prevents autoimmune diseases.

T cell populations produced in accordance with the present invention may be enriched with T cells that are specific to, i.e. target, truncal neo-antigens. That is, the T cell population that is produced in accordance with the present invention will have an increased number of T cells that target one or more truncal neo-antigens. For example, the T cell population produced in accordance with the invention will have an increased number of T cells that target a truncal neo-antigen compared with the T cells in the sample isolated from the subject. That is to say, the composition of the T cell population will differ from that of a "native" T cell population (i.e. a population that has not undergone the identification and expansion steps discussed herein), in that the percentage or proportion of T cells that target a truncal neo-antigen will be increased.

T cell populations produced in accordance with the present invention may be enriched with T cells that are specific to, i.e. target, truncal neo-antigens (i.e. clonal neo-antigens - as used herein the terms "truncal" neo-antigen and "clonal" neoantigen are equivalent, and the terms "branch" neo-antigen and "sub-clonal" neoantigen are equivalent), and may have a ratio of T cells that target truncal neoantigens to T cells that target branch neo-antigens which will be higher in favour of the T cells that target truncal neo-antigens as compared with T cells in the sample isolated from the subject.

That is, the T cell population that is produced in accordance with the present invention will have an increased number of T cells that target one or more truncal neo-antigens. For example, the T cell population of the invention will have an increased number of T cells that target a truncal neo-antigen compared with the T cells in the sample isolated from the subject. That is to say, the composition of the T cell population will differ from that of a "native" T cell population (i.e. a population that has not undergone the identification and expansion steps discussed herein), in that the percentage or proportion of T cells that target a truncal neo-antigen will be increased, and the ratio of T cells in the population that target truncal neo-antigens to T cells that target branch neo-antigens will be higher in favour of the T cells that target truncal neo-antigens.

The T cell population produced in accordance with the invention may have at least about 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or 100% T cells that target a truncal neo-antigen. For example, the T cell population may have about 0.2%-5%, 5%-10%, 10-20%, 20-30%, 30-40%, 40-50 %, 50-70% or 70-100% T cells that target a truncal neo-antigen. In one aspect the T cell population has at least about 1, 2, 3, 4 or 5% T cells that target a truncal neo-antigen, for example at least about 2% or at least 2% T cells that target a truncal neo-antigen.

Alternatively put, the T cell population may have not more than about 5, 10, 15, 20, 25, 30, 35, 40, 45, 50, 55, 60, 65, 70, 75, 80, 85, 90, 91, 92, 93, 94, 95, 96, 97, 98, 99, 99.1, 99.2, 99.3, 99.4, 99.5, 99.6, 99.7, 99.8% T cells that do not target a truncal neo-antigen. For example, the T cell population may have not more than about 95%-99.8%, 90%-95%, 80-90%, 70-80%, 60-70%, 50-60 %, 30-50% or 0-30% T cells that do not target a truncal neo-antigen. In one aspect the T cell population has not more than about 99, 98, 97, 96 or 95% T cells that do not target a truncal neo-antigen, for example not more than about 98% or 95% T cells that do not target a truncal neoantigen

An expanded population of truncal neo-antigen-reactive T cells may have a higher activity than a population of T cells not expanded, for example, using a truncal neoantigen peptide. Reference to "activity" may represent the response of the T cell population to restimulation with a truncal neo-antigen peptide, e.g. a peptide corresponding to the peptide used for expansion, or a mix of truncal neo-antigen peptides. Suitable methods for assaying the response are known in the art. For example, cytokine production may be measured (e.g. IL2 or IFNγ production may be measured). The reference to a "higher activity" includes, for example, a 1-5, 5-10, 10-20, 20-50, 50-100, 100-500, 500-1000-fold increase in activity. In one aspect the activity may be more than 1000-fold higher.

In a preferred embodiment the method of the invention provides a plurality or population, i.e. more than one, of T cells wherein the plurality of T cells comprises a T cell which recognises a clonal neo-antigen and a T cell which recognises a different clonal neoantigen. As such, the method of the invention provides a plurality of T cells which recognise different clonal neo-antigens. Different T cells in the plurality or population may alternatively have different TCRs which recognise the same truncal neo-antigen.

In a preferred embodiment the number of clonal neo-antigens recognised by the plurality of T cells is from 2 to 1000. For example, the number of clonal neo-antigens recognised may be 2, 3, 4, 5, 6, 7, 8, 9, 10, 20, 50, 100, 150, 200, 250, 300, 350, 400, 450, 500, 550, 600, 650, 700, 750, 800, 850, 900, 950 or 1000, preferably 2 to 100. There may be a plurality of T cells with different TCRs but which recognise the same clonal neo-antigen.

The T cell population may be all or primarily composed of CD8+ T cells, or all or primarily composed of a mixture of CD8+ T cells and CD4+ T cells or all or primarily composed of CD4+ T cells.

In particular embodiments, the T cell population is generated from T cells isolated from a subject with a tumour.

For example, the T cell population may be generated from T cells in a sample isolated from a subject with a tumour. The sample may be a tumour sample, a peripheral blood sample or a sample from other tissues of the subject.

In a particular embodiment the T cell population is generated from a sample from the tumour in which the truncal neo-antigen is identified. In other words, the T cell population is isolated from a sample derived from the tumour of a patient to be treated. Such T cells are referred to herein as 'tumour infiltrating lymphocytes' (TILs). T cells may be isolated using methods which are well known in the art. For example, T cells may be purified from single cell suspensions generated from samples on the basis of expression of CD3, CD4 or CD8. T cells may be enriched from samples by passage through a Ficoll-paque gradient.

Expansion of NES T cells may be performed using methods which are known in the art. For example, NES T cells may be expanded by *ex vivo* culture in conditions which are known to provide mitogenic stimuli for T cells. By way of example, the NES T cells may be cultured with cytokines such as IL-2 or with mitogenic antibodies such as anti-CD3 and/or CD28. The NES T cells may also be co-cultured with irradiated antigen-presenting cells (APCs), such as dendritic cells pulsed with peptides containing the identified truncal mutations as single stimulants or as pools of stimulating truncal neo-antigens or peptides.

Expansion of NES T cells may be performed using methods which are known in the art, including for example the use of artificial antigen presenting cells (aAPCs), for example which provide additional co-stimulatory signals, and autologous PBMCs which present appropriate peptides. By way of example, the autologous PBMCs may be pulsed with peptides containing truncal mutations as discussed herein as single stimulants, or alternatively as pools of stimulating truncal neo-antigen peptides.

Described herein is a method for producing a composition comprising an antigen presenting cell and a truncal neo-antigen or truncal neo-antigen peptide. The truncal neo-antigen may be identified according to methods described herein. In one aspect said method comprises the following steps:

(a) identifying a truncal neo-antigen in a tumour from a subject which comprises the steps of:

i) determining mutations present in a sample isolated from the tumour; and
ii) identifying a truncal mutation which is a mutation present in essentially all tumour cells; and
iii) identifying a truncal neo-antigen, which is an antigen encoded by a sequence which comprises the truncal mutation;

or

i) determining the mutations present in a plurality of samples isolated from a tumour ;
ii) identifying a truncal mutation which is a mutation present in all samples;
iii) identifying a truncal neo-antigen, which is an antigen encoded by a sequence which comprises the truncal mutation; and

b) producing a composition comprising said truncal neo-antigen or a truncal neo-antigen peptide and an antigen presenting cell.

Described herein is a composition comprising an antigen presenting cell, e.g. a dendritic cell, and a truncal neo-antigen or truncal neo-antigen peptide. The truncal neo-antigen may be identified according to the methods discussed herein.

The composition may be produced according to a method as described herein. The composition may also be used in the methods of the invention described herein, in methods of producing a T cell or T cell population or composition as discussed herein.

Compositions as described herein may be a pharmaceutical composition which additionally comprises a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

Described herein is a method for providing a T cell population which targets a truncal neo-antigen in a tumour which comprises the steps of:

i) identifying a T cell from a sample isolated from a subject which is capable of specifically recognising said truncal neo-antigen; and

ii) expanding the T cell to provide a T cell population which targets the truncal neo-antigen, wherein said T cell is expanded by co-culture with antigen presenting cells which present truncal neo-antigen peptides derived from said truncal neoantigen.

The resulting T cell population is enriched with T cells which target truncal neoantigens.

In one aspect the antigen presenting cells have been pulsed or loaded with said peptide.

Also described herein is a T cell composition which comprises a population of truncal neo-antigen-specific T cells, wherein said population of truncal neo-antigen-specific T cells are produced by co-culturing T cells with antigen presenting cells which present neo-antigen peptides.

In one aspect the antigen presenting cell is a dendritic cell. In one aspect the antigen presenting cell is irradiated.

In one aspect the antigen presenting cell is a cell capable of presenting the relevant peptide, for example in the correct HLA context. Such a cell may be an autologous activated PBMC expressing an autologous HLA molecule, or a non-autologous cell expressing an array of matched HLAs. In one aspect the artificial antigen presenting cell is irradiated.

NES T cells may also be enriched by initial stimulation of TILs with truncal neoantigens in the presence or absence of exogenous APCs followed by polyclonal stimulation and expansion with cytokines such as IL-2 or with mitogenic antibodies such as anti-CD3 and/or CD28. Such methods are known in the art. For example, see Forget et al. J Immunother. 2014 Nov-Dec;37(9):448-60, Donia et al. Cytotherapy. 2014 Aug;16(8):1117-20, Donia et al. Scand J Immunol. 2012 Feb;75(2):157-67 and Ye et al. J Transl Med. 2011 Aug 9;9:131.

Identification of NES T cells in a mixed starting population of T cells may be performed using methods which are known in the art. For example, NES T cells may be identified using MHC multimers comprising a truncal neo-antigen peptide identified by the method described herein.

MHC multimers are oligomeric forms of MHC molecules, designed to identify and isolate T-cells with high affinity to specific antigens amid a large group of unrelated T-cells. Multimers may be used to display class 1 MHC, class 2 MHC, or nonclassical molecules (e.g. CD1d).

The most commonly used MHC multimers are tetramers. These are typically produced by biotinylating soluble MHC monomers, which are typically produced recombinantly in eukaryotic or bacterial cells. These monomers then bind to a backbone, such as streptavidin or avidin, creating a tetravalent structure. These backbones are conjugated with fluorochromes to subsequently isolate bound T-cells via flow cytometry, for example.

Described herein is an MHC multimer comprising a truncal neo-antigen peptide. The truncal neo-antigen may be identified by a method as described herein.

Described herein is a method for producing an MHC multimer which may be used according to the invention as described herein. Said method comprises the steps of:

(a) identifying a truncal neo-antigen in a tumour from a subject which comprises the steps of:

i) determining mutations present in a sample isolated from the tumour; and

ii) identifying a truncal mutation which is a mutation present in essentially all tumour cells; and

iii) identifying a truncal neo-antigen, which is an antigen encoded by a sequence which comprises the truncal mutation;

or

i) determining the mutations present in a plurality of samples isolated from a tumour;

ii) identifying a truncal mutation which is a mutation present in all samples;

iii) identifying a truncal neo-antigen, which is an antigen encoded by a sequence which comprises the truncal mutation; and

(b) producing a truncal neo-antigen peptide from said truncal neo-antigen; and

(c) producing an MHC multimer comprising said truncal neo-antigen peptide.

MHC multimers may be used in methods for identifying, isolating, expanding or otherwise producing a T cell, T cell population or composition produced in accordance with the present invention. Truncal neo-antigen peptides may be synthesised using methods which are known in the art.

The term "peptide" is used in the normal sense to mean a series of residues, typically L-amino acids, connected one to the other typically by peptide bonds between the $\alpha$-amino and carboxyl groups of adjacent amino acids. The term includes modified peptides and synthetic peptide analogues.

The peptide may be made using chemical methods (Peptide Chemistry, A practical Textbook. Mikos Bodansky, Springer-Verlag, Berlin.). For example, peptides can be synthesized by solid phase techniques (Roberge JY et al (1995) Science 269: 202-204), cleaved from the resin, and purified by preparative high performance liquid chromatography (e.g., Creighton (1983) Proteins Structures And Molecular Principles, WH Freeman and Co, New York NY). Automated synthesis may be achieved, for example, using the ABI 43 1 A Peptide Synthesizer (Perkin Elmer) in accordance with the instructions provided by the manufacturer.

The peptide may alternatively be made by recombinant means, or by cleavage from the polypeptide which is or comprises the neo-antigen. The composition of a peptide may be confirmed by amino acid analysis or sequencing (e.g., the Edman degradation procedure).

The truncal neo-antigen peptide may comprise the cancer cell specific mutation/truncal mutation (e.g. the non-silent amino acid substitution encoded by a SNV) at any residue position within the peptide. By way of example, a peptide which is capable of binding to an MHC class I molecule is typically 7 to 13 amino acids in length. As such, the amino acid substitution may be present at position 1, 2, 3, 4, 5, 6, 7, 8, 9, 10, 11, 12 or 13 in a peptide comprising thirteen amino acids.

In a further aspect, longer peptides, for example 27-31 mers, may be used, and the mutation may be at any position, for example at the centre of the peptide, e.g. at positions 13, 14, 15 or 16 can also be used to stimulate both CD4+ and CD8+ cells to recognise clonal neo-antigens

Described herein is an MHC multimer comprising a truncal neo-antigen peptide as defined herein.

T CELL COMPOSITION

Further described is a T cell composition which comprises a truncal neo-antigen specific T cell.

The T cell composition may be a pharmaceutical composition comprising a plurality of neo-antigen specific T cells as defined herein. The pharmaceutical composition may additionally comprise a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion.

In a preferred embodiment of the present invention, the subject described herein is a mammal, preferably a human, cat, dog, horse, donkey, sheep, pig, goat, cow, mouse, rat, rabbit or guinea pig, but most preferably the subject is a human.

The methods may be used *in vitro, ex vivo* or *in vivo,* for example either for *in situ* treatment or for *ex vivo* treatment followed by the administration of the treated cells to the body.

As described herein the T cell or T cell population or composition is reinfused into a subject, for example following T cell isolation and expansion as described herein. Suitable methods to achieve this will be known to one skilled in the art. For example, methods for generating, selecting and expanding T cells are known in the art, see e.g. Dudley J Immunother. 2003; 26(4): 332-342, and Rosenberg et al. 2011 Clin Cancer Res:17(13):4550-7. Methods for reinfusing T cells are described, for example, in Dudley et al. Clin Cancer Res. 2010 Dec 15; 16(24): 6122-6131. 2011 and Rooney et al. Blood. 1998 Sep 1;92(5):1549-55.

The truncal neo-antigen specific T cell may be any T cell which is capable of recognising a truncal neo-antigen (i.e. a NES T cell).

The NES T cell is a T cell provided by a method of the present invention.

The NES T cell may be an engineered T cell. For example, the NES T cell may express a chimeric antigen receptor (CAR) or a T cell receptor (TCR) which specifically binds to a truncal neo-antigen or a truncal neo-antigen peptide (for example an affinity enhanced T cell receptor (TCR) which specifically binds to a truncal neo-antigen or a truncal neo-antigen peptide).

CARs are proteins which, in their usual format, graft the specificity of a monoclonal antibody (mAb) to the effector function of a T-cell. Their usual form is that of a type I transmembrane domain protein with an antigen recognizing amino terminus, a spacer, a transmembrane domain all connected to a compound endodomain which transmits T-cell survival and activation signals.

The most common form of these molecules use single-chain variable fragments (scFv) derived from monoclonal antibodies to recognize a target antigen. The scFv is fused via a spacer and a transmembrane domain to a signaling endodomain. Such molecules result in activation of the T-cell in response to recognition by the scFv of its target. When T cells express such a CAR, they recognize and kill target cells that express the target antigen. Several CARs have been developed against tumour associated antigens, and adoptive transfer approaches using such CAR-expressing T cells are currently in clinical trial for the treatment of various cancers.

Affinity-enhanced TCRs are generated by identifying a T cell clone from which the TCR $\alpha$ and $\beta$ chains with the desired target specificity are cloned. The candidate TCR then undergoes PCR directed mutagenesis at the complimentary determining regions of the α and β chains. The mutations in each CDR region are screened to select for mutants with enhanced affinity over the native TCR. Once complete, lead candidates are cloned into vectors to allow functional testing in T cells expressing the affinity-enhanced TCR.

NES T cells may bear high affinity TCRs, and hence affinity enhancement may not be necessary. High affinity TCRs may be isolated from NES T cells from a subject and may not require affinity enhancement.

Candidate T cell clones capable of binding a truncal neo-antigen peptide may be identified using the MHC multimers comprising the truncal neo-antigen peptide as described herein, for example.

Identified TCRs and/or CARs which specifically target a truncal neo-antigen peptide or truncal neo-antigen may be expressed in autologous T cells from a subject using methods which are known in the art, for example by introducing DNA or RNA coding for the TCR or CAR by one of many means including transduction with a viral vector, transfection with DNA or RNA.

The autologous T cells may be from a sample isolated from a subject as described herein.

In the context of the invention the T cell is an engineered T cell.

VACCINE

Described herein is a vaccine comprising a truncal neo-antigen or truncal neo-antigen peptide as defined herein. For example, the truncal neo-antigen or truncal neoantigen peptide may be identified by the method described herein. The vaccine may comprise more than one different truncal neo-antigen or truncal neo-antigen peptide, for example 2, 3, 4, 5, 6, 7, 8, 9 or 10 different peptides. The truncal neo-antigen may also be in the form of a protein.

The vaccine may comprise a polypeptide which comprises a truncal neo-antigen as defined herein. The vaccine may comprise more than one different polypeptide each comprising a truncal neo-antigen, for example 2, 3, 4, 5, 6, 7, 8, 9 or 10 different polypeptides.

The vaccine may be a pharmaceutical composition which additionally comprises a pharmaceutically acceptable carrier, diluent or excipient. The pharmaceutical composition may optionally comprise one or more further pharmaceutically active polypeptides and/or compounds. Such a formulation may, for example, be in a form suitable for intravenous infusion. See, for example, Butterfield, BMJ. 2015 22;350 for a discussion of cancer vaccines.

In particular, the vaccine may additionally comprise an adjuvant. Examples of adjuvants include but are not limited to aluminium salts, oil emulsions and bacterial components (e.g. LPS and liposomes).

Suitable doses of peptides in the vaccine may be determined by one skilled in the art. The dose may depend on the peptide which is to be used. For *in vivo* use of a peptide an *in vivo* dose of 0.1-4000μg, e.g. 0.1 -2000μg, 0.1 -1000 μg or 0.1 -500 μg, for example 0.1 -100μg, may be employed.

The vaccine as discussed herein may lead to generation of an immune response in the subject. An "immune response" which may be generated may be humoral and/or cell-mediated immunity, for example the stimulation of antibody production, or the stimulation of cytotoxic or killer cells, which may recognise and destroy (or otherwise eliminate) cells expressing antigens corresponding to the antigens in the vaccine on their surface. The term "stimulating an immune response" thus includes all types of immune responses and mechanisms for stimulating them and encompasses stimulating CTLs. Preferably the immune response which is stimulated is cytotoxic CD8+ T cells and helper CD4+T Cells. The extent of an immune response may be assessed by markers of an immune response, e.g. secreted molecules such as IL-2 or IFNy or the production of antigen specific T cells.

In addition, a truncal neo-antigen vaccine may be delivered in the form of a cell, such as an antigen presenting cell, for example as a dendritic cell vaccine. The antigen presenting cell such as a dendritic cell may be pulsed or loaded with the truncal neoantigen or truncal neo-antigen peptide or genetically modified (via DNA or RNA transfer) to express one, two or more truncal neo-antigens or truncal neo-antigen peptides (see e.g. Butterfield 2015 *supra*; Palucka 2013 *supra*), for example 2, 3, 4, 5, 6, 7, 8, 9 or 10 truncal neo-antigens or truncal neo-antigen peptides. Methods of preparing dendritic cell vaccines are known in the art.

Suitable vaccines may also be in the form of DNA or RNA vaccines relating to truncal neo-antigens or truncal neo-antigen peptides as described herein. For example, DNA or RNA encoding one or more truncal neo-antigen, or peptide or protein derived therefrom may be used as the vaccine, for example by direct injection to a subject. For example, DNA or RNA encoding 2, 3, 4, 5, 6, 7, 8, 9 or 10 truncal neo-antigens, or peptide or protein derived therefrom. The one or more truncal neo-antigen or truncal neo-antigen peptide may be delivered via a bacterial or viral vector containing DNA or RNA sequences which encode one or more truncal neo-antigens or truncal neo-antigen peptides.

Vaccines as described herein may be administered in any suitable way. For example, any suitable delivery mechanism as known in the art may be used. The vaccine may involve the use of a vector delivery system, or a vector delivery system may not be necessary. Vectors may be viral or bacterial. Liposomes may be used as a delivery system. Listeria vaccines or electroporation may also be used.

Described herein is a cell expressing a truncal neo-antigen or truncal neo-antigen peptide on its surface (or intracellularly), or a population of such cells, which cell or population is obtainable (or obtained) by methods as defined herein. The cell may be an antigen presenting cell such as a dendritic cell.

For *in vivo* administration of cells as described herein, any mode of administration of the cell population which is common or standard in the art may be used, e.g. injection or infusion, by an appropriate route. In one aspect $1 \times 10^4$ to $1 \times 10^8$ cells are administered per kg of subject (e.g. $1.4 \times 10^4$ to $2.8 \times 10^6$ per kg in human). In one aspect about or not more than $10^7$ cells per kg of subject are administered. Thus, for example, in a human, a dose of $0.1 - 20 \times 10^7$ cells per kg of subject may be administered in a dose, i.e. per dose, for example as a dose of T cells or a vaccination dose. In one aspect, between $1 \times 10^4$ to $1 \times 10^6$ cells, between $1 \times 10^6$ to $1 \times 10^6$ cells, between $1 \times 10^6$ to $1 \times 10^7$ cells or between $1 \times 10^7$ to $1 \times 10^8$ cells per kg of subject are administered. For vaccination applications, $1-20 \times 10^6$ cells per dose may be used. The dose can be repeated at later times if necessary.

The vaccine may be used in the treatment of cancer.

Described herein is a method for treating cancer in a subject comprising administering a vaccine as described herein to said subject. The method may additionally comprise the step of identifying a subject who has cancer.

Described herein is a method for producing a vaccine comprising a truncal neoantigen peptide or truncal neo-antigen, said method comprising the steps of:

(a) identifying a truncal neo-antigen in a tumour from a subject which comprises the steps of:

i) determining mutations present in a sample isolated from the tumour; and
ii) identifying a truncal mutation which is a mutation present in essentially all tumour cells; and
iii) identifying a truncal neo-antigen, which is an antigen encoded by a sequence which comprises the truncal mutation;

or

i) determining the mutations present in a plurality of samples isolated from a tumour;
ii) identifying a truncal mutation which is a mutation present in all samples;
iii) identifying a truncal neo-antigen, which is an antigen encoded by a sequence which comprises the truncal mutation; and

(b) producing a truncal neo-antigen peptide or truncal neo-antigen from said truncal neo-antigen; and
(c) producing a vaccine with said truncal neo-antigen peptide or truncal neo-antigen protein.

Producing the vaccine may involve preparing a dendritic cell vaccine, wherein said dendritic cell presents a truncal neoantigen or truncal neoantigen peptide.

A truncal neo-antigen protein may also be used in the vaccines and methods relating to vaccination.

Described herein is a method for producing a vaccine comprising a DNA or RNA molecule encoding a truncal neo-antigen peptide or truncal neo-antigen, said method comprising the steps of:

(a) identifying a truncal neo-antigen in a tumour from a subject which comprises the steps of:

i) determining mutations present in a sample isolated from the tumour; and
ii) identifying a truncal mutation which is a mutation present in essentially all tumour cells; and
iii) identifying a truncal neo-antigen, which is an antigen encoded by a sequence which comprises the truncal mutation;

or

i) determining the mutations present in a plurality of samples isolated from a tumour;
ii) identifying a truncal mutation which is a mutation present in all samples;
iii) identifying a truncal neo-antigen, which is an antigen encoded by a sequence which comprises the truncal mutation; and

(b) producing a DNA or RNA molecule encoding the truncal neo-antigen peptide or truncal neo-antigen; and
(c) producing a vaccine with said DNA or RNA molecule.

The vaccine may be delivered by suitable methods as described hereinbefore.

In one aspect the vaccination is therapeutic vaccination. In this aspect the vaccine is administered to a subject who has cancer to treat the cancer.

In a further aspect the vaccination is prophylactic vaccination. In this aspect the vaccine is administered to a subject who may be at risk of developing cancer.

In one aspect the vaccine is administered to a subject who has previously had cancer and in whom there is a risk of the cancer recurring.

A vaccine may also be in the form of DNA or RNA coding for one or several of the truncal neo-antigenic peptides or proteins and delivered by additional methods including but not limited to viral vectors, antigen presenting cells and electroporation.

CANCER

T cells which specifically target a truncal neo-antigen may be used in methods to treat cancer.

'To treat' relates to the therapeutic use of the T cell composition produced in accordance with the present invention. Herein the T cell composition may be administered to a subject having an existing disease or condition in order to lessen, reduce or improve at least one symptom associated with the disease and/or to slow down, reduce or block the progression of the disease.

The cancer may be, for example, bladder cancer, gastric, oesophageal, breast cancer, colorectal cancer, cervical cancer, ovarian cancer, endometrial cancer, kidney cancer (renal cell), lung cancer (small cell, non-small cell and mesothelioma), brain cancer (eg. gliomas, astrocytomas, glioblastomas), melanoma, lymphoma, small bowel cancers (duodenal and jejunal), leukemia, pancreatic cancer, hepatobiliary tumours, germ cell cancers, prostate cancer, head and neck cancers, thyroid cancer and sarcomas. In a preferred aspect of the invention the cancer is lung cancer, preferably non small-cell lung cancer. In another aspect of the invention the cancer is melanoma.

Treatment using the methods of the present invention may also encompass targeting circulating tumour cells and/or metastases derived from the tumour.

Treatment with the T cell composition produced in accordance with the present invention targeting one or more truncal neo-antigens may help prevent the evolution of therapy resistant tumour cells which often occurs with standard approaches.

The T cell compositions for treating cancer according to the present invention may be performed in combination with additional cancer therapies. In particular, the T cell compositions produced in accordance with the present invention may be administered in combination with checkpoint blockade therapy, co-stimulatory antibodies, chemotherapy and/or radiotherapy, targeted therapy or monoclonal antibody therapy.

Checkpoint inhibitors include, but are not limited to, PD-1 inhibitors, PD-L1 inhibitors, Lag-3 inhibitors, Tim-3 inhibitors, TIGIT inhibitors, BTLA inhibitors and CTLA-4 inhibitors, for example. Co-stimulatory antibodies deliver positive signals through immune-regulatory receptors including but not limited to ICOS, CD137, CD27 OX-40 and GITR. In a preferred embodiment the checkpoint inhibitor is a CTLA-4 inhibitor.

A chemotherapeutic entity as used herein refers to an entity which is destructive to a cell, that is the entity reduces the viability of the cell. The chemotherapeutic entity may be a cytotoxic drug. A chemotherapeutic agent contemplated includes, without limitation, alkylating agents, anthracyclines, epothilones, nitrosoureas, ethylenimines/methylmelamine, alkyl sulfonates, alkylating agents, antimetabolites, pyrimidine analogs, epipodophylotoxins, enzymes such as L-asparaginase; biological response modifiers such as IFN$\alpha$, IL-2, G-CSF and GM-CSF; platinum coordination complexes such as cisplatin, oxaliplatin and carboplatin, anthracenediones, substituted urea such as hydroxyurea, methylhydrazine derivatives including N-methylhydrazine (MIH) and procarbazine, adrenocortical suppressants such as mitotane (o,p'-DDD) and aminoglutethimide; hormones and antagonists including adrenocorticosteroid antagonists such as prednisone and equivalents, dexamethasone and aminoglutethimide; progestin such as hydroxyprogesterone caproate, medroxyprogesterone acetate and megestrol acetate; estrogen such as diethylstilbestrol and ethinyl estradiol equivalents; antiestrogen such as tamoxifen; androgens including testosterone propionate and fluoxymesterone/equivalents; antiandrogens such as flutamide, gonadotropin-releasing hormone analogs and leuprolide; and non-steroidal antiandrogens such as flutamide.

'In combination' may refer to administration of the additional therapy before, at the same time as or after administration of the T cell composition produced in accordance with the present invention.

In addition or as an alternative to the combination with checkpoint blockade, the T cell composition produced in accordance with the present invention may also be genetically modified to render them resistant to immune-checkpoints using gene-editing technologies including but not limited to TALEN and Crispr/Cas. Such methods are known in the art, see e.g. US20140120622. Gene editing technologies may be used to prevent the expression of immune checkpoints expressed by T cells including but not limited to PD-1, Lag-3, Tim-3, TIGIT, BTLA CTLA-4 and combinations of these. The T cell as discussed here may be modified by any of these methods.

The T cell produced in accordance with the present invention may also be genetically modified to express molecules increasing homing into tumours and or to deliver inflammatory mediators into the tumour microenvironment, including but not limited to cytokines, soluble immune-regulatory receptors and/or ligands.

The invention will now be further described by way of Examples, which are meant to serve to assist one of ordinary skill in the art in carrying out the invention and are not intended in any way to limit the scope of the invention, which, in the broadest sense, is as defined in the claims.

EXAMPLES

Example 1 - Identification of truncal neo-antigens in non-small cell lung cancer tumours

Tumour samples from a non-small cell lung cancer (NSCLC) tumour were subjected to deep exon sequence analysis to determine the extent of intra tumour heterogeneity (ITH), mutational load in each tumour region and to distinguish mutations present in all tumour cells from those present in only a subset. In parallel, single cell suspensions generated from the same tumour regions were processed, aliquoted and stored for later *in vitro* analysis and expansion.

*Identification of single nucleotide variants from exome sequencing data*

Exome sequencing was performed on multi region samples isolated from NSCLC tumours. Raw paired end reads (100bp) in FastQ format generated by the Illumina pipeline were aligned to the full hg19 genomic assembly (including unknown contigs) obtained from GATK bundle 2.8, using bwa mem (bwa-0.7.7) (Li and Durbin; 2009; Bioinformatics; 25(14):1754-60). Picard tools v1.107 was then applied to clean, sort and merge files from the same patient region and to remove duplicate reads (http://broadinstitute.github.io/picard). Quality control metrics were obtained using a combination of picard tools (1.107), GATK (2.8.1) and FastQC (0.10.1) (http://www.bioinformatics.babraham.ac. uk/projects/fastqc/).

SAMtools mpileup (0.1.16) (Li *et al.;* Bioinformatics; 2009; 25(16); 2078-2079) was used to locate non-reference positions in tumour and germ-line samples. Bases with a phred score of <20 or reads with a mapping-quality <20 were skipped. BAQ computation is disabled and the coefficient for downgrading mapping quality is set to 50.

Somatic single nucleotide variants (SNVs) between tumour and matched germ-line were determined using VarScan2 somatic (v2.3.6) (Koboldt et al.; Genome Res. 2012. 22: 568-576) utilizing the output from SAMtools mpileup. Default parameters were used with the exception of minimum coverage for the germ-line sample set to 10, minimum variant frequency is 0.01 and tumour purity 0.5. VarScan2 processSomatic was then used to extract the somatic variants.

The resulting SNV calls were filtered for false positives using Varscan2's associated fpfilter.pl script, having first run the data through bam-readcount (0.5.1). Additionally, further filtering was applied whereby variants were only be accepted if present in $\geq$ 5 reads and $\geq$ 5% variant allele frequency (VAF) in at least one tumour region with germ-line VAF $\leq$ 1%. If a variant was found to meet these criteria in a single region, then the VAF threshold was reduced to $\geq$ 1% in order to detect low frequency variants in other tumour regions.

*Copy number analysis*

Processed sample exome SNP and copy number data from paired tumor-normal was generated using VarScan2 (v2.3.6). Varscan2 copynumber was run using default parameters with the exception of min-coverage (8) and data-ratio. The data-ratio was calculated on a per-sample basis as described in Koboldt et al. (Genome Res.; 2012; 22: 568-576). Output from Varscan was then processed using the Sequenza R package 2.1.1 to provide segmented copy number data and cellularity and ploidy estimates for all samples based on the exome sequence data. The following settings were used: breaks.method = 'full', gamma = 40, kmin = 5, gamma.pcf = 200, kmin.pcf = 200.

*RNA-seq analysis*

Raw paired end reads are trimmed and aligned to the human reference genome and transcriptome using Tophat (version 1.3.3) (Trapnell et al.; 2009; Bioinformatics; 25(9):1105-11). Expression values were then calculated as fragments per kilobase of exone per million fragments mapped (FPKM) using Cufflinks (Trapnell et al.; 2010; Nat Biotech; 28(5); 511-5), with upper quartile normalization, fragment bias correction and multiread correction enabled.

*Identification of truncal SNVs*

The set of SNVs identified were classified as truncal or branched based on their cancer cell fraction (CCF) estimates in all tumour regions sequenced. In brief, the CCF, describing the proportion of cancer cells harbouring a mutation, is calculated by integrating copy number and purity estimates with variant allele frequencies.

For each variant, the expected variant allele frequency (VAF), given the CCF, was calculated as follows:

$$\text{VAF (CCF)} = p^{*}\text{CCF} / \text{CPN}_{norm} (1\text{-}p) + p^{*}\text{CPN}_{mut}$$

where $CPN_{mut}$ corresponds to the local copy number of the tumor, and p is the tumor purity, and $CPN_{norm}$ the local copy number of the matched normal sample.

For a given mutation with 'a' alternative reads, and a depth of 'N', the probability of a given CCF was estimated using a binomial distribution

$$P(CCF) = binom(a|N, VAF(CCF)).$$

CCF values were then calculated over a uniform grid of 100 CCF values (0.01,1) and subsequently normalized to obtain a posterior distribution.

Any SNV clonally present (CCF 95% confidence interval >= 0.75) in all tumour regions sequenced was considered truncal. Conversely, any SNV only present in a subset of tumour regions or with a CCF 95% confidence interval <= 0.75 in any region was considered branched (see Figure 2 for summary).

Example 2 - HLA Type Predictions

For each subject, germline whole exome sequencing FASTQ files were mapped to a reference FASTA file containing the sequences for known HLA alleles. Mapping was performed using Razers3 (Weese et al.; Bioinformatics; 2012; 28(20): 2592-2599) with a percent identity threshold of 90, a maximum of one hit, and a distance range of 0. Once mapped, the generated SAM files were converted to FASTQ and used as input to the Optitype prediction algorithm (Szolek et al.; Bioinformatics; 2014; 30(23):3310-3316) with default parameters. Optitype generates a predicted 4-digit resolution HLA type for each patient, which was stored for use in HLA-binding prediction.

*HLA Binding Predictions*

Coding mutations called from the tumour multi-region whole exome sequencing data were used to generate all possible 9-11mer mutant peptides from the neo-antigen, capturing the mutated amino acid in each position of the n-mer.

Thus, for a given SNV mutation, in total 446 peptides were produced. In addition, corresponding wildtype peptides were also generated.

The fasta file containing all mutant and wildtype peptide sequences and the predicted 4-digit HLA type were then used as input to NetMHC (Lundegaard et al.; 2008; Nucleic Acids Res; 36:W509-12), which predicts the binding affinity of each peptide to the patient's specific HLA alleles. Peptides that are predicted to bind at <500 nM were classified as putative weak neo-antigens, while those than bind at <50 nM were classified as putative strong neo-antigens. Moreover, for each peptide a delta score was calculated reflecting the difference in binding between mutant and wildtype peptides (see Figure 3).

Example 3 - Identification of putative truncal neo-antigens

All putative neo-antigens were classified as truncal or branched based on their cancer cell fraction in the tumour regions sequenced (as described in Example 1). Binding peptides that derive from a mutation found in every region of the tumour sequenced were identified as potential truncal neo-antigens (as described in Example 2).

*Filtering of putative truncal neo-antigens using RNAseq data*

All putative truncal neo-antigens were further filtered using RNA seq data. Specifically, the mean transcript length was used to convert from the calculated FPKM to TPM (transcripts per million) and identify putative truncal neo-antigen as those that are expressed at a median greater than 10 TPM.

Example 4 - Processing and expansion of tumour infiltrating Lymphocytes (TILs) from NSCLC samples

Tumors were minced under sterile conditions followed by enzymatic digestion (RPMI-1640 with Liberase TL research grade (Roche) and DNAse I (Roche)) at 37°C for 30 minutes before mechanical tissue separation using gentleMACS (Miltenyi Biotech). Resulting single cell suspensions were enriched for leukocytes by passage through a Ficoll-paque gradient. Live cells were counted and frozen at -80°C before transfer to liquid nitrogen. TILs were expanded using a rapid expansion protocol (REP) in T25 flasks containing EX-VIVO media supplemented with 10% human serum, soluble anti-CD3 (OKT3), 6000IU/mL recombinant human (rhIL-2) and $2x10^7$ irradiated PBMCs pooled from 3 allogeneic healthy donors. Once expansion was evident, fresh media containing rhIL-2 at 3000IU/mL was added every three days. Following 2 weeks of expansion, TILs were counted, phenotyped and frozen at -80°C before use in relevant assays or long-term storage in liquid nitrogen.

*Identification of Neo-antigen specific (NES) T cells using soluble peptide/HLA multimers*

The highest ranked neo-antigenic peptide sequences were synthetized (n=240) and used to generate fluorescently labelled, custom-made MHC multimers for the identification of NES T cells within expanded tumour-infiltrating lymphocytes (TILs). *In vitro* expanded TILs were stained with fluorescent custom-made MHC multimers loaded with the predicted mutated peptides or control Cytomegalovirus (CMV) peptides and analysed by multi-colour flow cytometry (see Figure 4).

Example 5 - *Ex vivo* and *in vivo* killing activity of NES T cells

Autologous tumour cell lines and expanded NES T cells are used to demonstrate the ability of these T cells to kill tumour targets *in vitro* and *in vivo*.

Tumour cell lines bearing trunk mutations and autologous expanded NES T cells are identified. A fixed number of tumour cells are plated on 96 well plates with varying numbers of NES T cells. *In vitro* killing activity of the NES T cells is evaluated at different time points (4-16 hours later following standard flow cytometry assays. For *in vivo* assays, immune deficient mice (NSG) are subcutaneously challenged with tumour cell lines and after engraftment are either left untreated or receive an i.v. infusion of 1-5x10$^6$ *in vitro* expanded autologous NES T cells.

Tumour growth in the treated and untreated groups is measured over time (every 3 days).

Example 6 - T cells targeting a clonal neo-antigen promote rejection of established heterogenous tumours whilst T cells targeting a subclonal neo-antigen fail to do so

To compare the *in vivo* anti tumour activity of T cells targeting clonal neo-antigens compared to those targeting subclonal neo-antigens we used a mouse model of melanoma (B16 line) and T cell receptor transgenic T cells (TCR Tg) recognising a clonal neo-antigen (trp1) or a subclonal neo-antigen (OTII).

T cells specific to Neo-antigens: CD4+Trp1 TCR Tg cells recognise a peptide derived from Tyrp1, an antigen present in all B16 melanoma cells. Trp1 TCR Tg cells are generated in mice lacking Tyrp1 hence, they recognise Tyrp1 as a neo-antigen.

CD4+OTII TCR Tg cells recognise a peptide derived from Ovalbumin (OVA), a model neo-antigen that can be artificially introduced by genetic engineering into B16 tumour cell lines.

To model clonal and subclonal neo-antigens we used mouse B16 melanoma cells (expressing Tyrp1) and B16-OVA cells also expressing Tyrp1 but in addition transduced to express OVA. By mixing these 2 cell lines we generate a model where Tyrp1 represents a clonal neo-antigen (expressed by all tumour cells) and OVA a subclonal neo-antigen (expressed only by some tumour cells).

Briefly B6 mice were injected at day 0 with a mixture of 12.5 x 10$^4$ B16 and 12.5 x 10$^4$ B16-OVA cells. At day 8 post tumour inoculation, and when tumours were palpable, mice were sub-lethally irradiated (5Gy) and received and intra venous (iv) infusion of either 30 x 10$^4$ CD4 OT-II cells (reactive to the subclonal neo-antigen) or 6 x 10$^4$ CD4 Trp-1 cells (reactive to the clonal neo-antigen), and 0.2 mg anti-CTLA-4 antibody intra peritoneal (i.p.). Mice received two additional doses of anti-CTLA-4 antibodies at day 11 and 14 (0.1 mg). As control, we used a group of mice challenged with tumour but left untreated.

The ability of OTII cells to reject established B16-OVA tumours was demonstrated in an additional group of mice inoculated only with 25 x 10$^4$ B16-OVA cells (in this case OVA is expressed by all B16-OVA cells in the tumour mass, hence representing a clonal neo-antigen). At day 8 mice were sublethally irradiated (5Gy), and treated with 30 x 10$^4$ CD4 OT-II cells i.v. and 0.2 mg anti-CTLA-4 antibody i.p. Mice received two additional doses of anti-CTLA-4 antibodies at day 11 and 14 (0.1 mg).

Results

In the control group, mice challenged with the heterogenous tumour mix (B16/B16-OVA) containing a clonal (tyrp1) and subclonal (OVA) neo-antigen and left untreated grew tumours and had to be sacrificed between days 20 and 30 after tumour challenge (Fig5A). Strikingly, all of the mice challenged with the B16/B16-OVA tumour mix but treated with TRP1 TCR Tg cells targeting a clonal neo-antigen were able to reject their tumours (Fig5B).

Conversely, when mice were treated with OTII TCR Tg T cells targeting a subclonal neo-antigen, none of the mice were able to reject their tumour (Fig1C). A slight delay in tumour progression was observed in this group suggesting potential control of B16-OVA cells expressing the subclonal neo-antigen, with eventual progression due to failure to reject tumour cells not expressing OVA (the subclonal neo-antigen) (Fig5C). Finally (Fig5D) demonstrates the ability of OTII TCRTg T cells to reject established tumours when all cells in the tumour express the OVA neo-antigen. Each line in each graph represents an independent mouse. 6 mice per groups were used for these experiments. (Fig 5E) shows all the experimental groups and the average tumour size in each group.

The data demonstrate the superior ability of T cells targeting clonal neo-antigen to reject established tumours compared to T cells only targeting subclonal neoantingens not expressed by all tumour cells within aneterogenous tumour mass.

**Claims**

1. A method for producing a T cell composition comprising an engineered T cell, wherein the T cell is engineered to express an introduced T cell receptor (TCR) which specifically binds a clonal neoantigen from a subject's cancer, wherein the method comprises the following steps:

    (a) identifying a clonal neoantigen-specific T cell from the subject which comprises the steps of:

        i) determining mutations present in one or more samples isolated from the subject's tumour;
        ii) identifying a clonal mutation, wherein a clonal mutation has a cancer cell fraction (CCF) estimate with a 95% confidence interval greater than or equal to 0.75 in each sample, wherein the CCF estimate in a sample is calculated by integrating variant allele frequencies, copy numbers and purity estimates for the sample;
        iii) identifying a clonal neoantigen, which is an antigen encoded by a sequence which comprises the clonal mutation;
        vi) identifying a T cell from a sample isolated from the subject which is capable of specifically recognising the clonal neoantigen as a clonal neoantigen-specific T cell;

    (b) isolating a TCR gene that encodes a TCR from the clonal neoantigen-specific T cell; and
    (c) engineering a T cell to express said TCR which recognises said clonal neoantigen to provide a T cell population which targets the clonal neoantigen; and
    (d) expanding the engineered T cell to provide a T cell composition.

2. A method according to claim 1 wherein the clonal neoantigen-specific T cell is identified from a sample of patient peripheral blood or tumour-infiltrating lymphocytes (TILs).

3. A method according to claim 1 or 2 wherein the clonal neoantigen-specific T cell is identified using a MHC multimer comprising the clonal neoantigen peptide.

4. A method according to any preceding claim which comprises introducing DNA or RNA coding for the TCR into the T cell by transduction with a viral vector or transfection with DNA or RNA.

5. A method according to any preceding claim wherein the TCR is an affinity-enhanced TCR which specifically binds a clonal neoantigen or a clonal neoantigen peptide.

6. A method according to any preceding claim wherein the population of T cells comprises CD8+ T cells, CD4+ T cells or CD8+ and CD4 T+ cells.

7. A method according to any preceding claim wherein the TCR is expressed in autologous T cells from the subject.

8. A T cell composition comprising an engineered T cell, wherein the T cell is engineered to express an introduced T cell receptor (TCR) which specifically binds a clonal neoantigen from a subject's cancer, for use in a method of treating said cancer in said subject, wherein the method comprises the following steps:

    (a) identifying a clonal neoantigen-specific T cell from the subject which comprises the steps of:

        i) determining mutations present in one or more samples isolated from the subject's tumour;
        ii) identifying a clonal mutation, wherein a clonal mutation has a cancer cell fraction (CCF) estimate with a 95% confidence interval greater than or equal to 0.75 in each sample, wherein the CCF estimate in a sample is calculated by integrating variant allele frequencies, copy numbers and purity estimates for the sample;
        iii) identifying a clonal neoantigen, which is an antigen encoded by a sequence which comprises the clonal mutation;

vi) identifying a T cell from a sample isolated from the subject which is capable of specifically recognising the clonal neoantigen as a clonal neoantigen-specific T cell;

(b) isolating a TCR gene that encodes a TCR from the clonal neoantigen-specific T cell; and
(c) engineering a T cell to express said TCR which recognises said clonal neoantigen to provide a T cell population which targets the clonal neoantigen; and
(d) expanding the engineered T cell to provide a T cell composition.

9. A T cell composition for use according to claim 8 wherein the cancer is non-small cell lung cancer (NSCLC) or melanoma.

10. A T cell composition for use according to claim 8 or 9 wherein the T cell composition is used in combination with a checkpoint inhibitor.

**Patentansprüche**

1. Verfahren zur Herstellung einer T-Zell-Zusammensetzung, die eine gentechnisch veränderte T-Zelle umfasst, wobei die T-Zelle gentechnisch verändert ist, um einen eingeführten T-Zell-Rezeptor (TCR) zu exprimieren, der spezifisch an ein klonales Neoantigen von einem Krebs eines Individuums bindet, wobei das Verfahren folgende Schritte umfasst:

(a) Identifizieren einer für das klonale Neoantigen spezifischen T-Zelle vom Individuum, das folgende Schritte umfasst:

i) Bestimmen von Mutationen, die in einer oder mehreren Proben vorhanden sind, die aus dem Tumor des Individuums isoliert wurden;
ii) Identifizieren einer klonalen Mutation, wobei eine klonale Mutation eine geschätzte Krebszellfraktion (CCF) mit einem Konfidenzintervall von 95 % von mehr als oder gleich 0,75 in jeder Probe aufweist, wobei die geschätzte CCF in einer Probe durch Integrieren von Allelvariantenhäufigkeiten, Kopienzahlen und Reinheitsschätzungen für die Probe berechnet wird;
iii) Identifizieren eines klonalen Neoantigens, bei dem es sich um ein Antigen handelt, für das eine Sequenz kodiert, welche die klonale Mutation umfasst;
vi) Identifizieren einer T-Zelle aus einer Probe, die vom Individuum isoliert wurde und welche Zelle in der Lage ist, das klonale Neoantigen spezifisch zu erkennen, als für das klonale Neoantigen spezifische T-Zelle;

(b) Isolieren eines TCR-Gens, das für einen TCR kodiert, von der für das klonale Neoantigen spezifischen T-Zelle; und
(c) gentechnisches Verändern einer T-Zelle, sodass sie den TCR exprimiert, der das klonale Neoantigen erkennt, um eine T-Zell-Population bereitzustellen, die auf das klonale Neoantigen abzielt; und
(d) Expandieren der gentechnisch veränderten T-Zelle, um eine T-Zell-Zusammensetzung bereitzustellen.

2. Verfahren nach Anspruch 1, wobei die für ein klonales Neoantigen spezifische T-Zelle aus einer Probe von peripherem Blut oder tumorinfiltrierenden Lymphozyten (TILS) eines Patienten identifiziert wird.

3. Verfahren nach Anspruch 1 oder 2, wobei die für ein klonales Neoantigen spezifische T-Zelle unter Verwendung eines MHC-Multimers identifiziert wird, welches das Peptid des klonalen Neoantigens umfasst.

4. Verfahren nach einem der vorangegangenen Ansprüche, welches das Einführen von DNA oder RNA, die für den TCR kodiert, in die T-Zelle durch Transduktion mit einem viralen Vektor oder Transfektion mit DNA oder RNA umfasst.

5. Verfahren nach einem der vorangegangenen Ansprüche, wobei der TCR ein TCR mit erhöhter Affinität ist, der spezifisch an ein klonales Neoantigen oder ein Peptid eines klonalen Neoantigens bindet.

6. Verfahren nach einem der vorangegangenen Ansprüche, wobei die Population von T-Zellen CD8+-T-Zellen, CD4+-T-Zellen oder CD8+- und CD4-T+-Zellen umfasst.

7. Verfahren nach einem der vorangegangenen Ansprüche, wobei der TCR in autologen T-Zellen vom Individuum exprimiert wird.

8. T-Zell-Zusammensetzung, die eine gentechnisch veränderte T-Zelle umfasst, wobei die T-Zelle gentechnisch verändert ist, um einen eingeführten T-Zell-Rezeptor (TCR) zu exprimieren, der spezifisch an ein klonales Neoantigen von einem Krebs eines Individuums bindet, zur Verwendung in einem Verfahren zur Behandlung des Krebses beim Individuum, wobei das Verfahren folgende Schritte umfasst:

(a) Identifizieren einer für das klonale Neoantigen spezifischen T-Zelle vom Individuum, das folgende Schritte umfasst:

i) Bestimmen von Mutationen, die in einer oder mehreren Proben vorhanden sind, die aus dem Tumor des Individuums isoliert wurden;
ii) Identifizieren einer klonalen Mutation, wobei eine klonale Mutation eine geschätzte Krebszellfraktion (CCF) mit einem Konfidenzintervall von 95 % von mehr als oder gleich 0,75 in jeder Probe aufweist, wobei die geschätzte CCF in einer Probe durch Integrieren von Allelvariantenhäufigkeiten, Kopienzahlen und Reinheitsschätzungen für die Probe berechnet wird;
iii) Identifizieren eines klonalen Neoantigens, bei dem es sich um ein Antigen handelt, für das eine Sequenz kodiert, welche die klonale Mutation umfasst;
vi) Identifizieren einer T-Zelle aus einer Probe, die vom Individuum isoliert wurde und welche Zelle in der Lage ist, das klonale Neoantigen spezifisch zu erkennen, als für das klonale Neoantigen spezifische T-Zelle;

(b) Isolieren eines TCR-Gens, das für einen TCR kodiert, von der für das klonale Neoantigen spezifischen T-Zelle; und
(c) gentechnisches Verändern einer T-Zelle, sodass sie den TCR exprimiert, der das klonale Neoantigen erkennt, um eine T-Zell-Population bereitzustellen, die auf das klonale Neoantigen abzielt; und
(d) Expandieren der gentechnisch veränderten T-Zelle, um eine T-Zell-Zusammensetzung bereitzustellen.

9. T-Zell-Zusammensetzung zur Verwendung nach Anspruch 8, wobei der Krebs nichtkleinzelliger Lungenkrebs (NSCLC) oder ein Melanom ist.

10. T-Zell-Zusammensetzung zur Verwendung nach Anspruch 8 oder 9, wobei die T-Zell-Zusammensetzung in Kombination mit einem Checkpoint-Inhibitor verwendet wird.

**Revendications**

1. Procédé de production d'une composition de lymphocytes T comprenant un lymphocyte T modifié génétiquement, dans lequel le lymphocyte T est modifié pour exprimer un récepteur de lymphocytes T (TCR) introduit qui se lie spécifiquement à un néoantigène clonal provenant du cancer d'un sujet, dans lequel le procédé comprend les étapes suivantes :

(a) identifier un lymphocyte T spécifique à un néoantigène clonal chez le sujet, qui comprend les étapes consistant à :

i) déterminer des mutations présentes dans un ou plusieurs échantillons isolés à partir de la tumeur du sujet ;
ii) identifier une mutation clonale, dans lequel une mutation clonale présente une estimation de fraction de cellules cancéreuses (CCF) avec un intervalle de confiance à 95 % supérieur ou égal à 0,75 dans chaque échantillon, dans lequel l'estimation de CCF dans un échantillon est calculée en intégrant des fréquences d'allèles variants, des nombres de copies et des estimations de pureté pour l'échantillon ;
iii) identifier un néoantigène clonal, qui est un antigène codé par une séquence qui comprend la mutation clonale ;
vi) identifier un lymphocyte T à partir d'un échantillon isolé à partir du sujet qui est capable de reconnaître spécifiquement le néoantigène clonal en tant que lymphocyte T spécifique à un néoantigène clonal ;

(b) isoler un gène TCR qui code un TCR à partir du lymphocyte T spécifique à un néoantigène clonal ; et
(c) modifier génétiquement un lymphocyte T pour exprimer ledit TCR qui reconnaît ledit néoantigène clonal pour fournir une population de lymphocytes T qui cible le néoantigène clonal ; et
(d) développer le lymphocyte T modifié pour fournir une composition de lymphocytes T.

**2.** Procédé selon la revendication 1, dans lequel le lymphocyte T spécifique à un néoantigène clonal est identifié à partir d'un échantillon de sang périphérique de patient ou de lymphocytes infiltrant une tumeur (TIL).

**3.** Procédé selon la revendication 1 ou 2, dans lequel le lymphocyte T spécifique à un néoantigène clonal est identifié en utilisant un multimère de CMH comprenant le peptide de néoantigène clonal.

**4.** Procédé selon l'une quelconque des revendications précédentes, qui comprend l'introduction d'ADN ou d'ARN codant pour le TCR dans le lymphocyte T par transduction avec un vecteur viral ou transfection avec de l'ADN ou de l'ARN.

**5.** Procédé selon l'une quelconque des revendications précédentes, dans lequel le TCR est un TCR à affinité améliorée qui se lie spécifiquement à un néoantigène clonal ou à un peptide de néoantigène clonal.

**6.** Procédé selon l'une quelconque des revendications précédentes, dans lequel la population de lymphocytes T comprend des lymphocytes T CD8+, des lymphocytes T CD4+ ou des lymphocytes T CD8+ et CD4+.

**7.** Procédé selon l'une quelconque des revendications précédentes, dans lequel le TCR est exprimé dans des lymphocytes T autologues provenant du sujet.

**8.** Composition de lymphocytes T comprenant un lymphocyte T modifié génétiquement, dans laquelle le lymphocyte T est modifié pour exprimer un récepteur de lymphocytes T (TCR) introduit qui se lie spécifiquement à un néoantigène clonal provenant du cancer d'un sujet, à utiliser dans un procédé de traitement dudit cancer chez ledit sujet, dans lequel le procédé comprend les étapes suivantes consistant à :

(a) identifier un lymphocyte T spécifique à un néoantigène clonal chez le sujet, qui comprend les étapes consistant à :

i) déterminer des mutations présentes dans un ou plusieurs échantillons isolés à partir de la tumeur du sujet ;
ii) identifier une mutation clonale, dans lequel une mutation clonale présente une estimation de fraction de cellules cancéreuses (CCF) avec un intervalle de confiance à 95 % supérieur ou égal à 0,75 dans chaque échantillon, dans lequel l'estimation de CCF dans un échantillon est calculée en intégrant des fréquences d'allèles variants, des nombres de copies et des estimations de pureté pour l'échantillon ;
iii) identifier un néoantigène clonal, qui est un antigène codé par une séquence qui comprend la mutation clonale ;
vi) identifier un lymphocyte T à partir d'un échantillon isolé à partir du sujet qui est capable de reconnaître spécifiquement le néoantigène clonal en tant que lymphocyte T spécifique à un néoantigène clonal ;

(b) isoler un gène TCR qui code un TCR à partir du lymphocyte T spécifique à un néoantigène clonal ; et
(c) modifier génétiquement un lymphocyte T pour exprimer ledit TCR qui reconnaît ledit néoantigène clonal pour fournir une population de lymphocytes T qui cible le néoantigène clonal ; et
(d) développer le lymphocyte T modifié pour fournir une composition de lymphocytes T.

**9.** Composition de lymphocytes T pour utilisation selon la revendication 8, dans laquelle le cancer est un cancer du poumon non à petites cellules (CPNPC) ou un mélanome.

**10.** Composition de lymphocytes T pour utilisation selon la revendication 8 ou 9, dans laquelle la composition de lymphocytes T est utilisée en combinaison avec un inhibiteur de point de contrôle.

FIGURE 1

Mutational heterogenity in NSCLC

Predicted Neo-antigenic landscape in NSCLC

Identification of neo-antigen reactive TILs by soluble peptide-HLA tetramer technology

FIGURE 2

FIGURE 3

Mutations identification by Exome seq and RNAseq

Predicted binding of patient's mutations to patient's HLA

Selection of 200 peptides with high predicted HLA binding affinity

Tetramer generation and identification of neoantigen-specific T cells

FIGURE 4

FIGURE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014168874 A

- US 20140120622 A

**Non-patent literature cited in the description**

- **BOA et al.** *Cancer Informatics*, 2014, vol. 13 (2), 67-82
- **ARES et al.** *Cold Spring Harb Protoc.*, 03 November 2014, vol. 2014 (11), 1139-48
- **KAMMERMEIER et al.** *J Med Genet.*, November 2014, vol. 51 (11), 748-55
- **YAP KL et al.** *Clin Cancer Res.*, 2014, vol. 20, 6605
- **MEYERSON et al.** *Nat. Rev. Genetics*, 2010
- **MARDIS.** *Annu Rev Anal Chem*, 2013
- **OBENAUS M et al.** *Nat Biotechnol.*, 2015, vol. 33 (4), 402-7
- **HADRUP.** *Nature Methods*, 2009, vol. 6, 520-526
- **ANDERSEN.** *Nature Protocol*, 2012, vol. 7, 891-902
- **LANDAU et al.** *Cell*, 14 February 2013, vol. 152 (4), 714-26
- **KOBOLDT et al.** *Genome Res*, 2012, vol. 22, 568-576
- **SZOLEK et al.** *Bioinformatics;*, 2014, vol. 30 (23), 3310-3316
- **LUNDEGAARD et al.** *Nucleic Acids Res.*, 2008, W509-12.2008
- *Bioinformatics.*, 01 June 2008, vol. 24 (11), 1397-8
- **SHEN et al.** *Proteome Sci.*, 07 November 2013, vol. 11 (1), S15
- **FORGET et al.** *J Immunother.*, November 2014, vol. 37 (9), 448-60
- **DONIA et al.** *Cytotherapy*, August 2014, vol. 16 (8), 1117-20

- **DONIA et al.** *Scand J Immunol.*, February 2012, vol. 75 (2), 157-67
- **YE et al.** *J Transl Med.*, 09 August 2011, vol. 9, 131
- **MIKOS BODANSKY.** Peptide Chemistry, A practical Textbook. Springer-Verlag
- **ROBERGE JY et al.** *Science*, 1995, vol. 269, 202-204
- **CREIGHTON.** Proteins Structures And Molecular Principles. WH Freeman and Co, 1983
- **DUDLEY.** *J Immunother*, 2003, vol. 26 (4), 332-342
- **ROSENBERG et al.** *Clin Cancer Res*, 2011, vol. 17 (13), 4550-7
- **DUDLEY et al.** *Clin Cancer Res.*, 15 December 2010, vol. 16 (24), 6122-6131
- **ROONEY et al.** *Blood*, 01 September 1998, vol. 92 (5), 1549-55
- **BUTTERFIELD.** *BMJ*, 2015, vol. 22, 350
- **LI ; DURBIN.** *Bioinformatics*, 2009, vol. 25 (14), 1754-60
- **KOBOLDT et al.** *Genome Res*, vol. 22, 568-576
- **TRAPNELL et al.** *Bioinformatics*, 2009, vol. 25 (9), 1105-11
- **TRAPNELL et al.** *Nat Biotech*, 2010, vol. 28 (5), 511-5
- **WEESE et al.** *Bioinformatics*, 2012, vol. 28 (20), 2592-2599
- **SZOLEK et al.** *; Bioinformatics;*, 2014, vol. 30 (23), 3310-3316
- **LUNDEGAARD et al.** *Nucleic Acids Res*, 2008, vol. 36, W509-12